(19) Europäisches Patentamt
European Patent Office
Office européen des brevets

(11) Veröffentlichungsnummer: **0 385 109**
**A2**

(12) # EUROPÄISCHE PATENTANMELDUNG

(21) Anmeldenummer: **90101544.6**

(22) Anmeldetag: **26.01.90**

(51) Int. Cl.[5]: **G02B 1/04, G11B 7/24, C07C 305/24, C07C 50/08, C07C 39/15, C07C 211/40, C07C 50/30**

(30) Priorität: **01.02.89 DE 3902939**

(43) Veröffentlichungstag der Anmeldung:
**05.09.90 Patentblatt 90/36**

(84) Benannte Vertragsstaaten:
**BE DE FR GB NL**

(71) Anmelder: **BASF Aktiengesellschaft**
**Carl-Bosch-Strasse 38**
**D-6700 Ludwigshafen(DE)**

(72) Erfinder: **Feuerherd, Karl-Heinz, Dr.**
**Kesselgraben 16**
**D-6719 Hettenleidelheim(DE)**
Erfinder: **Hedtmann-Rein, Carola, Dr.**
**Winzerweg 1**
**D-6945 Hirschberg(DE)**
Erfinder: **Leber, Ludger, Dr.**
**Mainstrasse 4**
**D-6701 Dannstadt-Schauernheim(DE)**

(54) **Formteil für optische Zwecke.**

(57) Die Erfindung betrifft ein neues, optisch klares, isotropes, von Orientierungsdoppelbrechung freies Formteil für optische Zwecke aus einem Gemisch aus Polyphenylenethern, Vinylaromatpolymerisaten und Zusatzstoffen. Das neue Formteil enthält in Spuren neue Diphenochinon- und/oder Diphenohydrochinonderivate als Zusatzstoffe. Daneben kann das neue Formteil neue Ammoniumsalze von Schwefelsäurehalbestern von Diphenohydrochinonen als weitere Zusatzstoffe enthalten. Das neue Formteil hat einen Gelbwert (YI, Yellowness Index) von 40 bis 90. Es läßt sich in einfacher Weise mit Hilfe von Spritzgußverfahren herstellen. Das neue Formteil in der Form einer runden gelochten Scheibe läßt sich hervorragend als dimensionsstabiles Substrat von Audio-Compact-Disks (CD), audiovisuellen Compact-Disks (CDV), laseroptischen Datenplatten und magneto-optischen Datenplatten verwenden, und Datenträger dieser Art, welche dieses neue dimensionsstabile Substrat enthalten, weisen besonders vorteilhafte anwendungstechnische Eigenschaften auf.

EP 0 385 109 A2

## Formteil für optische Zwecke

Die vorliegende Erfindung betrifft ein optisch klares, isotropes, von Orientierungsdoppelbrechung freies Formteil für optische Zwecke, welches aus einem neuen Gemisch aus Polyphenylenethern, Vinylaromatpolymerisaten und Zusatzstoffen besteht.

Optisch klare, isotrope, von Orientierungsdoppelbrechung freie Formteile für optische Zwecke aus einem Gemisch aus Polyphenylenethern und Vinylaromatpolymerisaten sind vom Stand der Technik her bekannt. So geht aus der US-A-4 373 065 eine laseroptische Datenplatte hervor, welche mindestens eine Schicht aus einem Gemisch aus Poly(2,6-dimethyl-phen-1,4-ylen-ether) und Polystyrol enthält. Dieses von Orientierungsdoppelbrechung freie Gemisch besteht aus 36 bis 40 Gew.-% an Poly(2,6-dimethyl-phen-1,4-ylen-ether) und aus 60 bis 64 Gew.-% an Polystyrol, wobei das jeweils verwendete Mischungsverhältnis vom mittleren Molekulargewicht des Polystyrols abhängig ist:

Um die Orientierungsdoppelbrechung des Polystyrols zu kompensieren, werden bei Polystyrol des mittleren Molekulargewichts von etwa 6,7 x $10^5$ in etwa 38 bis 40 Gew.-% bei Polystyrol des mittleren Molewkulargewichts von $10^5$ bis 3 x $10^5$ in etwa 36 bis 40 Gew.-% bei Polystyrol des zahlenmittleren Molekulargewichts von $10^5$ bis 1,1 x $10^5$ in etwa 37 bis 39 Gew.-% und bei Polystyrol des mittleren Molekulargewichts von $10^4$ in etwa 31 bis 33 Gew.-% an Poly(2,6-dimethyl-phen-1,4-ylen-ether) benötigt. Anstelle dieses Phenylenethers können auch die 2,6-Diethyl-, 2-Methyl-6-ethyl-, 2-Methyl-6-propyl-, 2,6-Dipropyl- oder die 2-Ethyl-6-propyl-substituierten Polyphenylenether verwendet werden. Des weiteren kommen anstelle von Polystyrol auch Poly(α-methylstyrol) oder Copolymerisate aus p-Chlorstyrol und o-Chlorstyrol in Betracht.

Aus der EP-A 0 225 801 geht ein Formteil für optische Zwecke hervor, welches im wesentlichen aus Vinylaromatpolymerisaten und Polyphenylenethern besteht, wobei die Polyphenylenether eine in Chloroform bei 25°C gemessene intrinsische Viskosität [$\eta$] von 0,3 bis 0,7 haben. Um von Orientierungsdoppelbrechung freie Gemische zu erzeugen, werden hierbei 30 bis 70 Gew.-%, vorzugsweise 40 bis 55 Gew.-%, des Vinylaromatpolymerisats mit 70 bis 30 Gew.-% insbesondere 60 bis 45 Gew.-% an Polyphenylenethern abgemischt. Die in dieser Weise hergestellten, optisch klaren, isotropen, von Orientierungsdoppelbrechung freien Gemische lassen sich durch Spritzgußverfahren zu Formteilen für optische Zwecke, insbesondere zu dimensionsstabilen Substraten von laseroptischen Datenplatten verarbeiten.

Des weiteren geht aus der JP-A-63-089335 ein Verfahren zur Herstellung eines optisch klaren, isotropen, von Orientierungsdoppelbrechung freien Formteils für optische Zwecke hervor, bei welchem man dem zu verarbeitenden Gemisch aus Polyphenylenethern und Vinylaromatpolymerisaten organische Phosphorverbindungen und/oder sterisch gehinderte Phenole als Stabilisatoren zusetzt. Hierdurch werden die innere Spannung und die Orientierungsdoppelbrechung verringert, Zersetzungserscheinungen bei der Verarbeitung werden unterdrückt, und die optische Transmission wird verbessert.

Aus der JP-A-63-056832 geht eine magneto-optische Datenplatte hervor, deren Substrat aus einer Mischung aus Vinylaromatpolymerisaten und Polyphenylenethern besteht. Geeignete Vinylaromaten zum Aufbau dieser Polymerisate sind Styrol, m-Methylstyrol, p-Methylstyrol, o-Chlorstyrol oder p-Chlorstyrol. Die Vinylaromatpolymerisate können noch andere Monomere wie Methacrylnitril, n-Propylmethacrylat, n-Butylmethacrylat, Maleinsäureanhydrid oder N-Phenylmaleinimid einpolymerisiert enthalten. Diese dimensionsstabilen Substrate weisen eine gute Adhäsionskraft gegenüber den ihnen aufliegenden Schutzschichten auf.

Aus der JP-A-63-013722 geht ein Spritzgußverfahren für die Herstellung eines dimensionsstabilen Substrates aus Vinylaromatpolymerisaten und Polyphenylenethern hervor. Bei diesem Verfahren wird das Gemisch aus Vinylaromatpolymerisaten und Polyphenylenethern bei einer Massetemperatur von 300 bis 340°C spritzgegossen.

In der deutschen Patentanmeldung P 37 27 093.1 wird eine laseroptische Datenplatte beschrieben, deren dimensionsstabiles Substrat aus einem optisch klaren, isotropen, homogenen, von Orientierungsdoppelbrechung freien Gemisch aus Poly(2,6-dimethyl-phen-1,4-ylen-ether) und Styrolpolymerisaten besteht. Die hierbei verwendeten Styrolpolymerisate enthalten, bezogen auf die Styrolpolymerisate, 0,1 bis 8 Gew.-% an einpolymerisiertem Acrylnitril, 1 bis 60 Gew.-% an einpolymerisiertem α-Methylstyrol, 1 bis 60 Gew.-% an einpolymerisiertem p-Methylstyrol und/oder 0,1 bis 10 Gew.-% an einpolymerisierten (Meth)Acrylaten. Diese zusätzlich vorhandenen Monomereinheiten können statistisch verteilt in den Polymerhauptketten des Polystyrols vorliegen. Sie können aber auch in der Form von einheitlichen oligomeren oder polymeren Blöcken vorliegen, welche entweder in die Polymerhauptketten des Polystyrols eingebaut und/oder als seitenständige Reste mit diesen Polymerhauptketten verknüpft sind. Des weiteren können die zusätzlichen Monomereinheiten oligomere oder polymere Homo- oder Copolymerisate oder Block- oder Propfmischpolymerisate bilden, die separat hergestellt und dann dem Polystyrol hinzugefügt werden. Darüber hinaus

können sowohl das α-Methylstyrol als auch das p-Methylstyrol die Monomereinheiten von oligomeren statistischen Copolymerisaten bilden, welche separat hergestellt und dann dem Polystyrol hinzugefügt werden. Unabhängig davon, in welcher Weise das Styrolpolymerisat modifiziert ist, enthält das von Orientierungsdoppelbrechung freie Gemisch stets nicht weniger als 40 Gew.-% an einpolymerisiertem Styrol. Diese Gemische lassen sich mit Hilfe von Spritzgußverfahren gut zu dimensionsstabilen Substraten für laseroptische Datenplatten verarbeiten, und dimensionsstabile Substrate, welche man aus diesen Gemischen hergestellt hat, weisen eine gute Spurrillenabformung, eine gute Planlage und eine besonders niedrige Orientierungsdoppelbrechung auf.

Trotz der bisher erzielten Fortschritte weisen die optisch klaren, isotropen, von Orientierungsdoppelbrechung freien Formteile für optische Zwecke aus Gemischen aus Polyphenylenethern, Vinylaromatpolymerisaten und gegebenenfalls Zusatzstoffen, insbesondere aber die aus diesen Gemischen hergestellten dimensionsstabilen Substrate laseroptischer Datenplatten, noch immer Nachteile auf, welche eine stetige Weiterentwicklung und Verbesserung notwendig machen. So haben die bisher bekannten dimensionsstabilen Substrate aus Gemischen aus Vinylaromatpolymerisaten und Polyphenylenethern oftmals ein Signal-Rausch-Verhältnis zur Folge, das den Anforderungen der Praxis nicht gerecht wird. Darüber hinaus kommt es bei der Herstellung der dimensionsstabilen Substrate durch den Spritzguß häufig zur Bildung von Stippen oder Gelteilchen; in manchen Fällen bilden sich sogar Verkohlungsteilchen. Des weiteren zeigen die laseroptischen und magneto-optischen Datenplatten, welche diese bekannten dimensionsstabilen Substrate enthalten, eine vergleichweise hohe Lesefehlerrate (bit error rate). Außerdem kann es bei laseroptischen Datenplatten, welche amorphe, thermisch veränderbare, Farbstoffe enthaltende Aufzeichnungsschichten aufweisen, zu einer Zerstörung der Aufzeichnungsschicht durch UV-Strahlung kommen, was dem Signal-Rausch-Verhältnis und der Lesefehlerrate der betreffenden laseroptischen Datenplatten abträglich ist. Darüber hinaus muß den bislang bekannten dimensionsstabilen Substraten zu Zwecken der Identifikation noch immer ein Farbstoff beigemengt werden, was stets zu Verarbeitungsproblemen und/oder zu Problemen der Verträglichkeit führt.

Aufgabe der vorliegenden Erfindung ist es, ein neues, optisch klares, isotropes, von Orientierungsdoppelbrechung freies Formteil für optische Zwecke aus einem Gemisch aus Polyphenylenethern, Vinylaromatpolymerisaten und Zusatzstoffen zu finden, welches die Nachteile des Standes der Technik nicht mehr länger aufweist. Dieses neue Formteile für optische Zwecke soll sich insbesondere mit Vorteil als dimensionsstabiles Substrat von Audio-Compact-Disks (CD), von audio-visuellen Compact-Disks (CDV), von laseroptischen Datenplatten und von magneto-optischen Datenplatten eignen.

Überraschenderweise wird diese Aufgabe durch ein neues, optisch klares, isotropes, von Orientierungsdoppelbrechung freies Formteil für optische Zwecke gelöst, welches aus einem Gemisch aus Polyphenylenethern, Vinylaromatpolymerisaten und Zusatzstoffen besteht, wobei als Zusatzstoffe geringe Mengen an neuen Diphenochinon- und/oder Diphenohydrochinon-Derivaten verwendet werden.

Gegenstand der vorliegenden Erfindung ist demnach ein optisches klares, isotropes, von Orientierungsdoppelbrechung freies Formteil für optische Zwecke aus einem Gemisch aus Polyphenylenethern, Vinylaromatpolymerisaten und Zusatzstoffen, welches dadurch gekennzeichnet ist, daß das Gemisch, bezogen auf seine Gesamtmenge, 1 bis 1000 ppm mindestens eines Zusatzstoffes der allgemeinen Formel I,

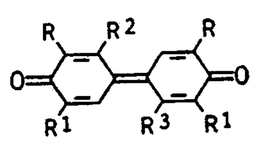

(I)

und/oder der allgemeinen Formel II,

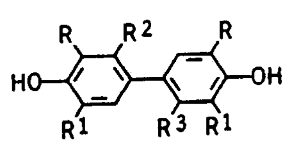

(II)

enthält, worin die Reste R und $R^1$ Wasserstoff-, Chlor- oder Bromatome, substituierte oder unsubstituierte $C_1$- bis $C_6$-Alkylgruppen, $C_3$- bis $C_6$-Alkenylgruppen, substituierte oder unsubstituierte $C_6$- bis $C_{18}$-Arylgruppen oder $C_1$- bis $C_6$-Alkoxygruppen bezeichnen, wobei die Reste R und $R^1$ gleich oder verschieden sein können und wobei

a) die Reste R für Wasserstoffatome oder Methylgruppen stehen, sofern die Reste $R^1$ Chlor- oder

Bromatome bezeichnen,

b) die Reste R für Methylgruppen stehen, sofern die Reste $R^1$ Isopropylgruppen bezeichnen, und wobei

c) die tert.-Butylgruppe ausgenommen ist; und worin die Reste $R^2$ und $R^3$ Wasserstoffatome, Hydroxygruppen oder $N(R^4)_2$-Gruppen bezeichnen, wobei

d) der Rest $R^3$ nur dann für ein Wasserstoffatom steht, wenn der Rest $R^2$ eine Hydroxygruppe oder eine $N(R^4)_2$-Gruppe bezeichnet,

e) der Rest $R^3$ nur dann für eine $N(R^4)_2$-Gruppe steht, wenn der Rest $R^2$ die selbe Bedeutung hat, und

f) die beiden Reste $R^4$ an einem Stickstoffatom gleich oder voneinander verschieden sein können und verzweigte, unverzweigte oder cyclische Alkylgruppen oder verzweigte, unverzweigte oder cyclische Aza- oder Oxaalkanylgruppen bezeichnen oder wobei die beiden Reste $R^4$ über eine zweiwertige Gruppe cyclisch miteinander verknüpft sind.

Außerdem wurden neue Derivate des Diphenochinons und Diphenohydrochinons gefunden, welche sich hervorragend als Zusatzstoffe für Gemische aus Vinylaromatpolymerisaten und Polyphenylenethern eignen.

Im folgenden wird das optisch klare, isotrope, von Orientierungsdoppelbrechung freie Formteil für optische Zwecke aus einem Gemisch aus Polyphenylenethern, Vinylaromatpolymerisaten und Zusatzstoffen der Kürze halber als "erfindungsgemäßes Formteil" bezeichnet.

Die erfindungswesentlichen Bestandteile der erfindungsgemäßen Formteile sind die neuen Zusatzstoffe der allgemeinen Formel I und/oder II.

Die erfindungsgemäßen Formteile können jeweils einen dieser neuen Zusatzstoffe I oder II enthalten. Sie können aber auch jeweils mehrere neue Zusatzstoffe I oder II enthalten. Des weiteren können die erfindungsgemäßen Formteile eine Mischung aus mindestens einem neuen Zusatzstoff I und mindestens einem neuen Zusatzstoff II enthalten.

Die neuen Zusatzstoffe I und/oder II sind in den erfindungsgmäßen Formteilen in Spuren enthalten. Unter Spuren wird hierbei eine Menge von 1 bis 1000 ppm, bezogen auf die Gesamtmenge des Gemischs, welches das erfindungsgemäße Formteil aufbaut, verstanden. Selbstverständlich können die neuen Zusatzstoffe I und/oder II in den erfindungsgemäßen Formteilen in größeren Mengen vorhanden sein. Indessen entfalten die neuen Zusatzstoffe I und/oder II ihre vorteilhafte technische Wirkung bereits in vollem Umfang bei einem Gehalt von 1 bis 1000 ppm, so daß sich eine weitere Erhöhung der Zuschlagsmenge an und für sich erübrigt.

Beispiele geeigneter Reste R und $R^1$ in den allgemeinen Formeln I und II sind Wasserstoff-, Chlor- oder Bromatome, Methyl-, Ethyl-, Propyl-, Isopropyl-, n-Butyl-, sek.-Butyl-, n-Pentyl- oder n-Hexylgruppen, Chlormethyl-, 2-Chlorethyl- oder 3-Chlorpropylgruppen, Prop-2-en-1-yl-, But-3-en-1-yl, Pent-4-en-1-yl-, 3-Methyl-but-2-en-1-yl- oder Hex-5-en-1-yl-gruppen, Phenyl-, Napht-1-yl-, Napht-2-yl-, 2′-Methyl-phen-1-yl-, 4′-Methyl-phen-1-yl-, 2′-Phenyl-phen-1-yl-, 3′-Phenyl-phen-1-yl-, 4′-tert.-Butyl-phen-1-yl- oder Terphenylylgruppen oder Methoxy-, Ethan-1-oxy-, Propan-1-oxy-, n-Butan-1-oxy-, n-Pentan-1-oxy- oder n-Hexan-1-oxygruppen.

Hierbei können die Reste R und $R^1$ in den neuen Zusatzstoffen I und/oder II gleich oder voneinander verschieden sein, wobei indes die folgenden beiden Einschränkungen gelten:

(a) stehen die Reste $R^1$ für Chlor- oder Bromatome, sind die Reste R Wasserstoffatome oder Methylgruppen, und

(b) stehen die Reste $R^1$ für Isopropylgruppen, so sind die Reste R Methylgruppen; und wobei

(c) tert. -Butylgruppen als Reste R und $R^1$ ausgenommen sind.

Bevorzugte Reste R und $R^1$ in den allgemeinen Formeln I und/oder II sind Methyl- oder Methoxygruppen, von denen die Methylgruppen ganz besonders bevorzugt sind.

Beispiele geeigneter Reste $R^2$ und $R^3$ in den allgemeinen Formeln I und II sind Wasserstoffatome, Hydroxygruppen oder $N(R^4)_2$-Gruppen. Hierbei können die Reste $R^2$ und $R^3$ gleich oder voneinander verschieden sein, wobei allerdings die folgenden Randbedingungen einzuhalten sind:

(d) steht der Rest $R^2$ für eine Hydroxygruppe oder eine $N(R^4)_2$-Gruppe, ist der Rest $R^3$ ein Wasserstoffatom, und

(e) für den Fall, daß der Rest $R^3$ für eine $N(R^4)_2$Gruppe steht, kann der Rest $R^2$ gleichfalls eine $N(R^4)_2$-Gruppe sein.

Die beiden Reste $R^4$, welche mit ein und demselben Stickstoffatom verknüpft sind, können gleich oder voneinander verschieden sein. Als Reste $R^4$ kommen verzweigte, unverzweigte oder cyclische Alkylgruppen oder verzweigte, unverzweigte oder cyclische Aza- oder Oxaalkanylgruppen in Betracht. Außerdem können die beiden Reste $R^4$ über eine zweiwertige Gruppe cyclisch miteinander verknüpft sein, so daß sie zusammen mit dem Stickstoffatom einen Heterocyclus bilden.

Beispiele geeigneter Reste $R^4$ sind Methyl-, Ethyl-, Propyl-, n-Butyl-, sek.-Butyl-, tert.-Butyl-, n-Pentyl-, tert.-Pentyl-, Neopentyl-, n-Hexyl-, n-Heptyl-, n-Octyl-, 2-Ethylhexyl-, Isooctyl-, tert.-Octyl-, n-Nonyl-, n-Decyl-, 5,5-Dimethylhexyl-, 6,6-Dimethylheptyl-, 7,7-Dimethyloctyl-, 8,8-Dimethylheptyl-, 2,4,4-Trimethylpentyl-, 3,5,5-Trimethylhexyl-, 4,6,6-Trimethyl-2-(1,3,3-trimethylbutyl)-heptyl-, 4,5,7,7-Tetramethyl-2-(1,3,3-trimethylbutyl)-heptyl-, 8,8-Dimethyl-4,5-bis(3,3-dimethylbutyl)-nonyl-, 5-Hydroxyheptyl-, 3-Aza-but-1-yl, 3-Aza-pent-1-yl-, 3-Aza-hex-1-yl-, 3-Aza-4,4-dimethyl-pent-1-yl-, 3-Aza-5,5-dimethyl-hex-1-yl, 3-tert.-Butyloxypropyl-, 4-tert.-Butyloxypentyl-, 6-tert.-Butyloxyhexyl-, 7-tert.-Butyloxyheptyl-, 3-tert.-Butyloxy-2-methylpropyl-, 3-tert.-Butyloxy-2,3-dimethylpropyl, 3-tert.-Butyloxy-2-methyloctyl-, 5,5-Bis(tert.-butyloxy)-3,4-dimethylpentyl-, 2-(2-Oxa-3,3-dimethylbutyl)-octyl-, 2-Ethyl-4-(3-oxa-4,4-dimethylpentyl)-8-oxa-9,9-dimethyldecyl-, 4-(1,3-Dioxan-2-yl)-butyl-, 4-(1,3-Dioxan-2-yl)-3-methylbutyl-, 4-(1,3-Dioxan-2-yl)-4-methylbutyl-, 4-(1,3-Dioxan-2-yl)-2,3-trimethylbutyl-, 5-(1,3-Dioxan-2-yl)-pentyl-, 6-(1,3-Dioxolan-2-yl)-hexyl-, 4-(4,4-Dimethyl-1,3-dioxacyclooctan-2-yl)-butyl, 4-(1,3-Dioxolan-2-yl)-3-methylbutyl- oder 6-(5,5-Dimethyl-1,3-dioxan-2-yl-)-3-ethyl-4,5-dimethylhexyl-Gruppen.

Die beiden Reste $R^4$ an ein und demselben Stickstoffatom können aber auch über eine zweiwertige Gruppe cyclisch miteinander verknüpft sein, so daß insgesamt die $N(R^4)_2$-Gruppe einen heterocyclischen Rest darstellt.

Beispiele geeigneter heterocyclischer Reste, welche sich aus einer solchen Verknüpfung der beiden Reste $R^4$ miteinander ergeben, sind Morpholin-1-yl-, Imidazol-1-yl-, $\Delta^2$-Imidazolin-1-yl-, Pyrrolidin-1-yl-, Piperid-1-yl-, Piperazin-1-yl-, 4-Methyl-piperazin-1-yl, Indolin-1-yl- oder Isoindolin-1-yl-Gruppen, von denen Piperid-1-yl- und die Morpholin-1-yl-Gruppe besonders bevorzugt sind.

Ganz besonders bevorzugte Reste $R^4$ sind Methyl-, Ethyl-, Propyl-, n-, Butyl- oder 3-Aza-4,4-dimethyl-pent-1-yl-Gruppen, von denen die n-Butyl-Gruppen einen herausragenden technischen Effekt bewirken.

Beispiele geeigneter Zusatzstoffe I sind die Zusatzstoffe I - 1 bis I - 18:

I - 1

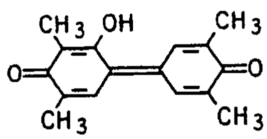

,

I - 2

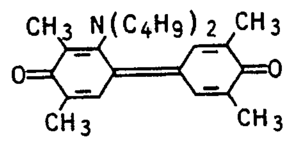

,

I - 3

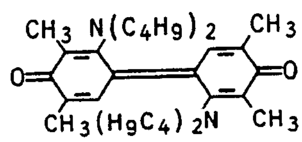

,

I - 4

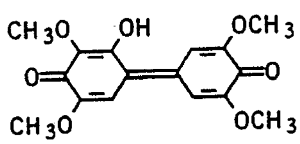

,

I - 5

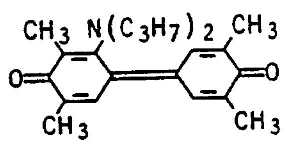

,

I - 6

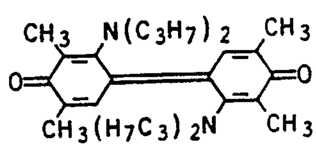

I - 7

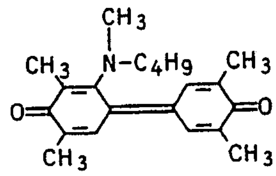

,

I - 8

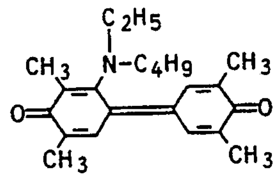

,

I - 9

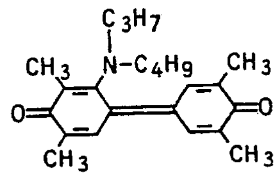

,

I - 10

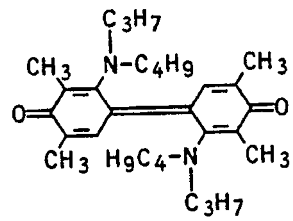

,

I - 11

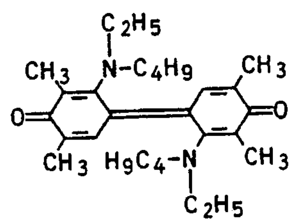

,

I - 12

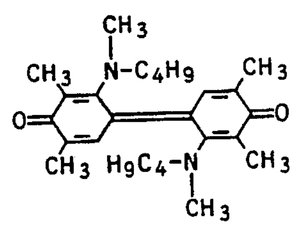

,

I - 13

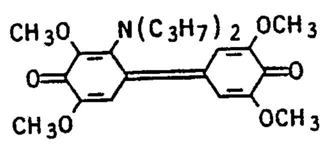

,

I - 14

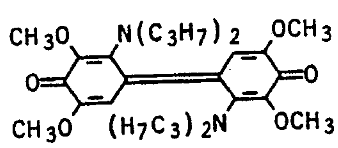

,

I - 15

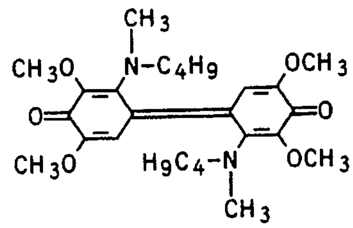

,

I- 16

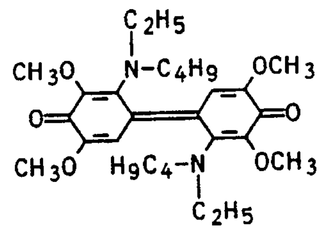

,

I - 17

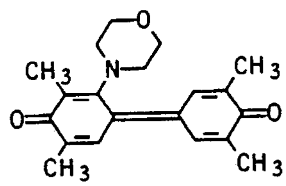

und

I - 18

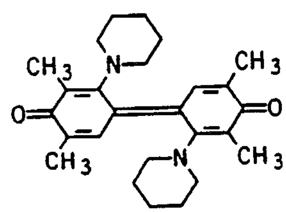

.

Von diesen sind die neuen Zusatzstoffe I - 1 und I - 3 ganz besonders bevorzugt.

Beispiele geeigneter neuer Zusatzstoffe II sind die Zusatzstoffe II - 1 bis II - 18

II - 1

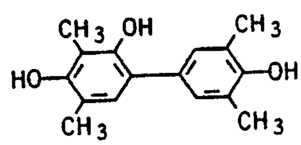

,

II - 2

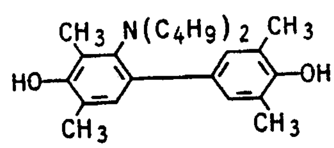

,

II - 3

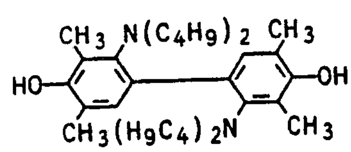

,

II - 4

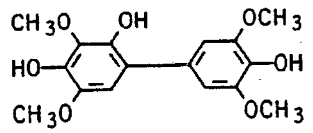

,

II - 5

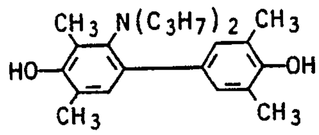

,

II - 6

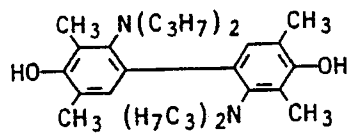

,

II - 7

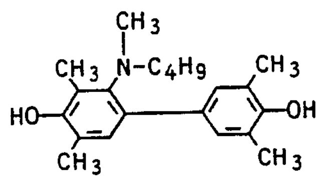

,

II - 8

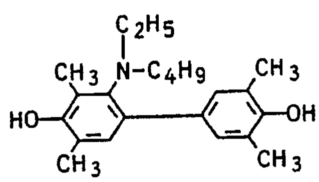

,

II - 9

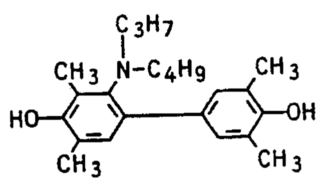

,

II - 10

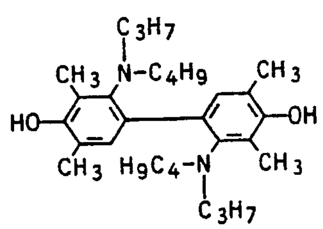

,

II - 11

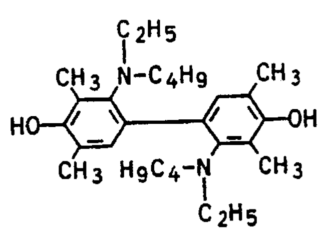

,

II - 12

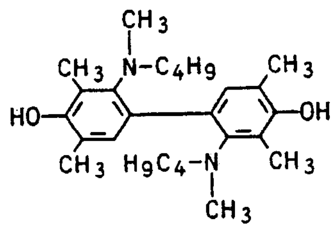

,

II - 13

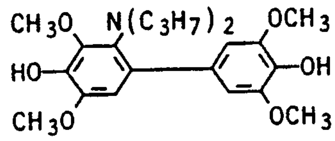

,

II - 14

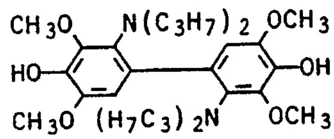

,

II - 15

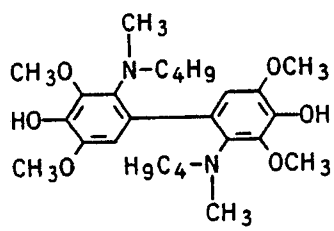

,

II - 16

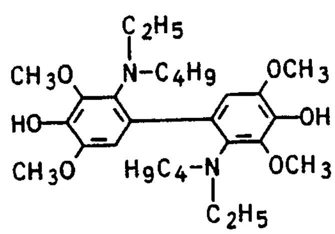

,

II - 17

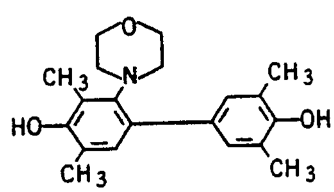

und

II - 18

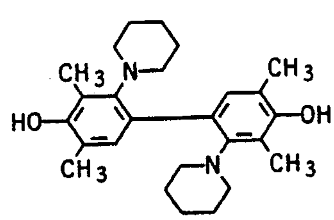

.

Von diesen ist der Zusatzstoff II - 2 ganz besonders bevorzugt.

Bei der Herstellung der neuen Zusatzstoffe I wird von Diphenochinonen der allgemeinen Formel IV ausgegangen,

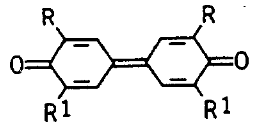

(IV)

worin die Reste R und $R^1$ die vorstehend angegebene Bedeutung haben. Die Herstellung dieser Diphenochinone IV ist an sich bekannt und erfolgt beispielsweise durch die Kupfer(II)-katalysierte Oxidation von disubstituierten Phenolen der allgemeinen Formel VII

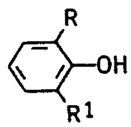

(VII)

in Acetonitril. Die in dieser Weise erhaltenen Diphenochinone IV werden dann beispielsweise nach den Methoden, wie sie von Wanzlick, Lehmann-Horchler, Mohrmann, Gritzki, Heidepriem und Pankow in dem Artikel "Neuere Methoden der Präparativen Organischen Chemie IV, Synthesen mit naszierenden Chinonen", Angewandte Chemie, Band 76, Seiten 313 bis 356, insbesondere Seite 315, 1964, für einkernige Chinone beschrieben sind, zu den neuen Zusatzstoffen I umgesetzt.

Die neuen Zusatzstoffe II werden aus den neuen Zusatzstoffen I durch Reduktion mit einem milden Reduktionsmittel in der Gegenwart von Wasser erhalten.

Außer den neuen Zusatzstoffen I und/oder II kann das erfindungsgemäße Formteil bzw. das Gemisch, aus dem das erfindungsgemäße Formteil besteht, bezogen auf seine Gesamtmenge, 1 bis 1000 ppm mindestens eines weiteren neuen Zusatzstoffs der allgemeinen Formel III enthalten,

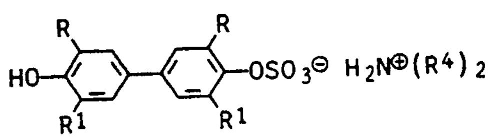

(III)

worin die Reste R, $R^1$ und $R^4$ die vorstehend angegebene Bedeutung haben. Zwar kann das erfindungsgemäße Formteil bzw. das Gemisch aus dem es besteht, mehr als 1000 ppm mindestens eines neuen Zusatzstoffs III enthalten, indessen entfalten diese Zusatzstoffe ihre vorteilhafte technische Wirkung bereits nach Zusatz geringer Mengen, so daß sich ein höherer Gehalt erübrigt.

Beispiele geeigneter neuer Zusatzstoffe III sind die neuen Zusatzstoffe III - 1 bis III - 10

III - 1

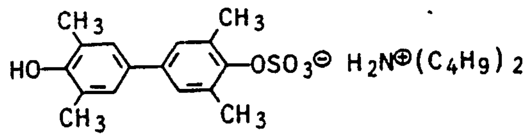

III - 2

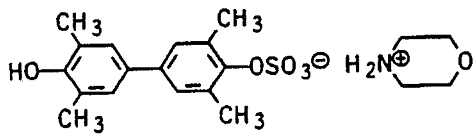
,

III - 3

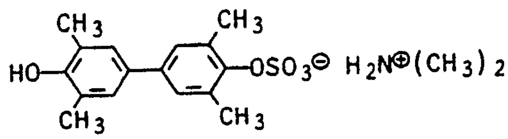
,

III - 4

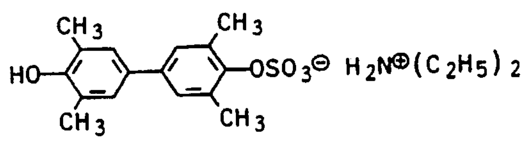
,

III - 5

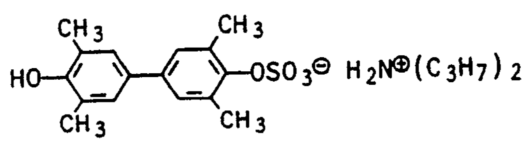
,

III - 6

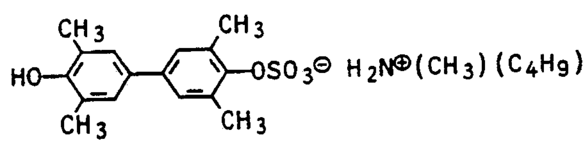
,

III - 7

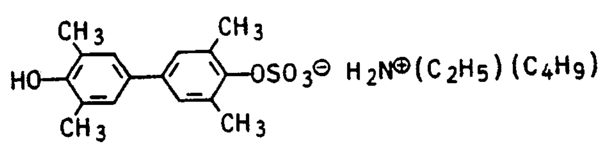
,

III - 8

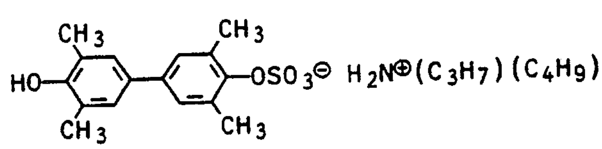
,

III - 9

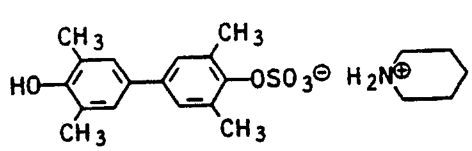
und

III - 10

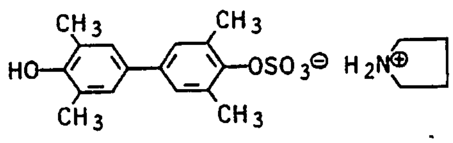

Von diesen ist der Zusatzstoff III - 1 ganz besonders bevorzugt.

Die Herstellung der neuen Zusatzstoffe III erfolgt durch Umsetzung der Diphenochinone IV in Toluol mit einer konzentrierten, wäßrigen, sauren Natriumsulfitlösung in der Gegenwart eines Amins der allgemeinen Formel VIII,

$NH(R^4)_2$ (VIII)

worin die beiden Reste $R^4$ die vorstehend angegebene Bedeutung haben. Hiernach werden die neuen Zusatzstoffe III aus dem Reaktionsgemisch mit Hilfe üblicher und bekannter präparativer Methoden isoliert und anschließend identifiziert.

Neben den neuen Zusatzstoffen I und/oder II sowie gegebenenfalls III kann das erfindungsgemäße Formteil bzw. das Gemisch aus dem es besteht, noch weitere Zusatzstoffe enthalten.

Beispiele für weitere geeignete Zusatzstoffe sind Zusatzstoffe der allgemeinen Formel IV

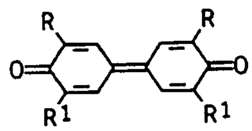

(IV)

und/oder der allgemeinen Formel V

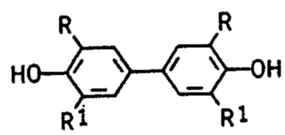

(V)

worin die Reste R und $R^1$ die vorstehend angegebene Bedeutung haben. Die Zusatzstoffe IV und V können in den erfindungsgemäßen Formteilen bzw. in den Gemischen, aus denen sie bestehen, in einer Gesamtmenge von 1 bis 10.000 ppm enthalten sein. Hierbei ist es von Vorteil, wenn der Gehalt an Zusatzstoffen IV, sofern sie verwendet werden, bei 1 bis 100 ppm liegt, wogegen der Gehalt an Zusatzstoffen V bis zu 10.000 ppm betragen kann.

Des weiteren kann das erfindungsgemäße Formteil bzw. das Gemisch, aus dem es besteht, 10 bis 10.000 ppm an oligomeren Phenylenethern der allgemeinen Formel VI enthalten,

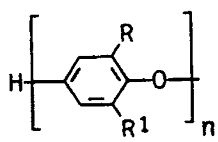

(VI)

worin die Reste R und $R^1$ die vorstehend angegebene Bedeutung haben und n eine ganze Zahl von 3 bis 15 ist.

Oligomere Phenylenether VI sind bekannte Verbindungen, welche beispielsweise über die bekannte Umverteilungsreaktion von disubstituierten Phenolen VII mit Polyphenylenethern, die Umsetzung der hierbei entstandenen Reaktionsprodukte zu Silylethern oder Acetaten und anschließender destillativer Auftrennung und Verseifung der betreffenden Silylether oder Acetate oder durch den gezielten Aufbau nach der Ullmann-Reaktion hergestellt werden können.

Die erfindungsgemäßen Formteile bzw. die Gemische, aus denen sie bestehen, sind für Licht einer Wellenlänge $\lambda$ <400 nm undurchlässig und für Licht einer Wellenlänge $\lambda$ >400 nm durchlässig. Sie haben einen Gelbwert YI (Yellowness Index) nach DIN 6167 von 40 bis 90, vorzugsweise 50 bis 80 und insbesondere 55 bis 70. Das Gemisch, aus welchem das erfindungsgemäße Formteil besteht, hat eine in 0,5 %iger Chloroformlösung bei 25 °C gemessene intrinsische Viskosität [$\eta$] von 0,5 bis 0,8, vorzugsweise

0,55 bis 0,75 und insbesondere 0,6 bis 0,75.

Das erfindungsgemäße Formteil besteht überwiegend aus Vinylaromatpolymerisaten und Polyphenylenethern. Dabei können sowohl die Vinylaromatpolymerisate als auch die Polyphenylenether als separat voneinander hergestellte und nachträglich miteinander vermischte Polymere im Gemisch bzw. im erfindungsgemäßen Formteil vorliegen. Indes kann auch ein Teil oder die Gesamtmenge der Vinylaromatpolymerisate und Polyphenylenether in der Form von Blockmischpolymeren oder Kammpolymeren miteinander verknüpft sein.

Unabhängig davon, ob die Vinylaromatpolymerisate und die Polyphenylenether als separat hergestellte und nachträglich miteinander vermischte Polymere oder als Blockmischpolymere oder Kammpolymere in dem erfindungsgemäßen Formteil vorliegen, liegt das Gewichtsverhältnis von Polyphenylenethern zu Vinylaromatpolymerisaten bei 30 : 70 bis 50 : 50, insbesondere 35 : 65 bis 40 : 60.

Vorteilhafte Polyphenylenether haben eine in 0,5 %iger Chloroformlösung bei 25 °C gemessene intrinsische Viskosität von 0,5 bis 0,6. Sie weisen eine Uneinheitlichkeit bezüglich des Molekulargewichts von $\overline{M}_w/\overline{M}_n$ von 4 bis 11, insbesondere 5 bis 10 auf. Besonders vorteilhafte Polyphenylenether haben ein zahlenmittleres Molekulargewicht $\overline{M}_n$ von 3500 bis 7000, insbesondere 4000 bis 6000. Weitere besonders vorteilhafte Polyphenylenether sind solche, welche ein massenmittleres Molekulargewicht $\overline{M}_w$ von 30.000 bis 50.000, insbesondere 35.000 bis 45.000 aufweisen. Ganz besonders vorteilhafte Polyphenylenether weisen sowohl das vorstehend genannte zahlenmittlere Molekulargewicht $\overline{M}_n$ und das vorstehend genannte massenmittlere Molekulargewicht $\overline{M}_w$ auf.

Beispiele geeigneter Polyphenylenether, welche die vorstehend genannten Bedingungen erfüllen, sind Polyphenylenether der allgemeinen Formel IX,

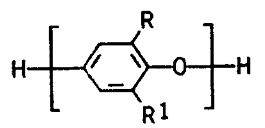

(IX)

worin die Reste R und $R^1$ die vorstehend angegebene Bedeutung haben, wie etwa Poly(2,6-dimethyl-phen-1,4-ylen-ether),
Poly(2,6-diethyl-phen-1,4-ylen-ether),
Poly(2,6-diphenyl-phen-1,4-ylen-ether),
Poly(2,6-diethoxy-phen-1,4-ylen-ether),
Poly(2-methyl-6-chlor-phen-1,4-ylen-ether),
Poly(2-methyl-6-brom-phen-1,4-ylen-ether),
Poly(2-methyl-6-isopropyl-phen-1,4-ylen-ether),
Poly(2,6-dimethoxy-phen-1,4-ylen-ether),
Poly(2-methyl-6-methoxy-phen-1,4-ylen-ether),
Poly(2-methyl-6-ethoxy-phen-1,4-ylen-ether) oder
Poly[2,6-bis(3-methyl-but-2-en-1-yl)-phen-1,4-ylen-ether], von denen Poly(2,6-dimethyl-phen-1,4-ylen-ether) besonders gut geeignet ist.

Die vorstehend genannten Polyphenylenether können untergeordneten Mengen, d.i. 0,001 bis 5 mol%, an in 2,3,6-Stellung trisubstituierten Phen-1,4-ylen-ether-Einheiten einpolymerisiert enthalten, wie etwa 2,3,6-Trimethyl-phen-1,4-ylen-ether-Einheiten.

Weitere wesentliche Bestandteile des erfindungsgemäßen Formteils bzw. des Gemischs, aus dem es besteht, sind Vinylaromatpolymerisate. Dabei kann es sich um Vinylaromathomo- oder -copolymerisate handeln, welche durch die bekannte radikalisch initiierte Polymerisation hergestellt worden sind. Es können aber auch Vinylaromathomo-, -co- und -Blockmischpolymerisate verwendet werden, welche nach den üblichen und bekannten anionischen Polymerisationsmethoden hergestellt worden sind.

Die für die Herstellung solcher Vinylaromatpolymerisate verwendeten Monomere sind üblich und bekannt. Besonders gut geeignet sind Styrol, α-Methylstyrol, o-, m-, oder p-Methylstyrol. Besonders vorteilhaft ist es, hochmolekulare Vinylaromatpolymerisate mit einem massemittleren Molekulargewicht $\overline{M}_w$ von $8 \times 10^4$ bis $2 \times 10^6$ gemeinsam mit niedermolekularen Vinylaromatpolymerisaten eines massenmittleren Molekulargewichts $\overline{M}_w$ von $5 \times 10^3$ bis $3 \times 10^4$ zu verwenden.

Die Vinylaromatpolymerisate können, bezogen auf ihre Gesamtmenge, 0,1 bis 8 Gew.% an Cyanoethylengruppen und/oder 0,01 bis 10 Gew.% an Gruppen der allgemeinen Formel X enthalten,

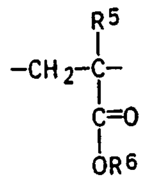

(X)

worin der Rest $R^5$ ein Wasserstoffatom oder eine Methylgruppe bezeichnet und der Rest $R^6$ für eine substituierte oder eine unsubstituierte $C_1$- bis $C_{10}$-Alkyl-, $C_5$- bis $C_8$-Cycloalkyl- oder $C_6$- bis $C_{10}$-Arylgruppe steht. Gruppen dieser Art leiten sich von den üblichen und bekannten Acryl- oder Methacrylsäureestern ab, welche sich besonders gut mit Vinylaromaten copolymerisieren lassen, wie etwa die Methyl-, Ethyl-, Propyl-, Isopropyl-, n-Butyl-, sek.-Butyl-, tert.-Butyl-, n-Pentyl-, n-Hexyl-, n-Heptyl-, n-Octyl-, 2-Ethylhexyl-, Nonyl-, Decyl-, Cyclopentyl-, Cyclohexyl-, 2-Methylcyclohexyl-, 2,4-Dimethylcyclohexyl-, Phenyl-, 4-Methylphenyl- oder die Naphtylester der Acrylsäure oder der Methacrylsäure.

Darüber hinaus können die Vinylaromatpolymerisate zusätzlich zu den Cyanoethylengruppen und/oder den Gruppen x oder anstelle von diesen, bezogen auf ihre Gesamtmenge, 0,01 bis 10 Gew.% an Gruppen der allgemeinen Formel XI enthalten,

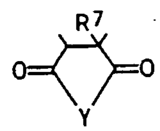

(XI)

worin $R^7$ ein Wasserstoffatom oder eine Methylgruppe bezeichnet und Y für ein Sauerstoffatom oder für eine N-$C_1$- bis N-$C_6$-Alkyl- oder eine N-$C_6$- bis N-$C_{10}$-Aryliminogruppe steht. Diese Gruppen XI leiten sich von Monomeren wie Malein- oder Itaconsäureanhydrid oder N-Methyl-, N-Ethyl-, N-Propyl-, N-Butyl-, N-Pentyl-, N-Hexyl-, N-Phenyl-, oder N-Naphtylmalein- oder -itaconimid ab, welche sich bekanntermaßen hervorragend zur Copolymerisation mit Vinylaromaten eignen.

Die Cyanoethylengruppen, die Gruppen X und/oder die Gruppen XI können als statistisch verteilte Monomereinheiten in den Vinylaromatpolymerisat-Hauptketten vorliegen. Sie können aber auch einheitliche oligomere oder polymere Blöcke bilden, welche in die Vinylaromatpolymerisat-Hauptketten eingebaut und/oder als seitenständige Reste mit diesen Hauptketten verknüpft sein können. Die genannten Gruppen können aber auch die Monomereinheiten von oligomeren oder polymeren Homo- oder Copolymerisaten oder von Block- oder Pfropfmischpolymerisaten bilden, welche separat hergestellt und dann den Vinylaromatpolymerisaten hinzugefügt wurden. Außerdem können die genannten Gruppen Polymerketten bilden, welche seitenständige Vinylaromatpolymerisatketten tragen.

Unabhängig davon, in welcher Form die Cyanoethylengruppen, die Gruppen X und/oder die Gruppen XI in dem erfindungsgemäßen Formteil enthalten sind, gelten die oben genannten Gewichtsbereiche für diese Gruppen. Beispiele besonders gut geeigneter Vinylaromatpolymerisate sind Polystyrol, Poly-α-methylstyrol, Poly-p-methylstyrol, Copolymerisate aus Styrol und α-Methylstyrol, Copolymerisate aus Styrol und p-Methylstyrol und Copolymerisate aus Styrol, α-Methylstyrol und p-Methystyrol oder Styrol-Methylmethacrylat-Copolymerisate.

Vinylaromatpolymerisate der vorstehend genannten Art, welche gegebenenfalls die Cyanoethylen-Gruppen, die Gruppen X und/oder die Gruppen XI enthalten, sind üblich und bekannt.

Das erfindungsgemäße Formteil bzw. das Gemisch, aus dem es besteht, kann, bezogen auf seine Gesamtmenge, 0,05 bis 10 Gew.% an Zusatzstoffen wie Antioxidantien, Weichmacher, Schmiermittel und Farbstoffe enthalten. Zusatzstoffe dieser Art sind üblich und bekannt. Geeignet sind vor allem diejenigen, welche sich in dem Gemisch molekulardispers verteilen lassen und Licht der Wellenlänge λ von 720 bis 1000 nm nur schwach oder gar nicht absorbieren.

Beispiele besonders gut geeigneter Zusatzstoffe dieser Art sind tert.-Butylkresol, Weißöl und Zinkstearat.

Das besonders vorteilhafte erfindungsgemäße Formteil enthält in der Hauptsache 30 bis 50, insbesondere 35 bis 40 Gew.-Teile Poly(2,6-dimethyl-phen-1,4-ylen-ether) auf 50 bis 70, insbesondere 60 bis 65 Gew.-Teile einer Mischung aus, bezogen auf die Mischung, 68 bis 97 Gew.% Polystyrol einem massenmittleren Molekulargewichts $\overline{M}_w$ von 8 x $10^4$ bis $10^6$; 3 bis 25 Gew.% eines statistischen Copolymerisat, bestehend aus 2 bis 98 Gew.% an einpolymerisiertem α-Methylstyrol und 98 bis 2 Gew.% an einpolymerisiertem p-Methylstyrol eines massenmittleren Molekulargewichts $\overline{M}_w$ von 7 x $10^3$ bis 2 x $10^4$. Außerdem

enthält das besonders vorteilhafte erfindungsgemäße Formteil stets mindestens einen der neuen Zusatzstoffe I - 1, I - 3 und II - 2 in einer Menge von 1 bis 1000 ppm.

Außerdem kann das erfindungsgemäße Formteil die neuen Zusatzstoffe III, insbesondere aber den neuen Zusatzstoff III - 1 in einer Menge von 1 bis 1000 ppm enthalten.

Das erfindungsgemäße Formteil weist zahlreiche besondere Vorteile auf. Insbesondere eignet es sich für optische Zwecke, bei denen es vor allem auf optische Klarheit, Isotropie, und Orientierungsdoppelbrechungsfreiheit von Formteilen ankommt. Hierbei kann das erfindungsgemäße Formteil eine beliebige äußere Form aufweisen, welche sich unmittelbar aus seinem Verwendungszweck ergibt. Aufgrund seiner besonderen vorteilhaften Eigenschaften kann das erfindungsgemäße Formteil vor allem als Träger digitaler oder analoger Daten dienen. Hierbei hat es die Form einer runden gelochten Scheibe eines Durchmessers von 50 bis 300 mm.

Hierbei kann das erfindungsgemäße Formteil in der Form einer runden gelochten Scheibe zwei planare Oberflächen aufweisen. Indes kann mindestens eine dieser beiden Oberflächen eine Reliefstruktur im Mikrometer- und/oder im Submikrometerbereich haben. Erfindungsgemäße Formteile bzw. runde gelochte Scheiben dieser Art werden vor allem als dimensionstabile Substrate von Audio-Compact-Disks (CD), audiovisuellen Compact-Disks (CDV), laseroptischen Datenplatten und magneto-optischen Datenplatten verwendet. Die erfindungsgemäßen Formteile in der Form solcher runder gelochter Scheiben werden im folgenden als "erfindungsgemäße dimensionstabile Substrate" bezeichnet.

Ganz besonders vorteilhafte erfindungsgemäße dimensionstabile Substrate haben eine Oberfläche mit einer Reliefstruktur im Mikrometer- und/oder Submikrometerbereich.

Diese Reliefstrukturen dienen der exakten Führung von Laserstrahlen, mit welchen die in den betreffenden Audio-Compact-Disks (CD), den audiovisuellen Compact-Disks (CDV), den laseroptischen Datenplatten und den magneto-optischen Datenplatten enthaltenen digitalen Daten gelesen werden. Die Reliefstrukturen gewährleisten ein rasches und genaues Ansprechen der Spurlagenservo- und Autofokussiereinrichtungen in den laseroptischen Schreib- und Leseköpfen der Plattenlaufwerke wie beispielsweise in CD-Plattenspielern. Die Reliefstrukturen ermöglichen oder verbessern deshalb das sogenannte "tracking". Außerdem können die Reliefstrukturen selbst Daten sein, wie dies beispielsweise bei den Audio-Compact-Disks (CD) und den audiovisuellen Compakt-Disks (CDV) der Fall ist. Die Reliefstrukturen können aber auch der Codierung von Daten dienen, welche mit Hilfe von Schreiblaserstrahlen in die Aufzeichnungsschichten der laseroptischen und magneto-optischen Datenplatten eingeschrieben werden.

Grundsätzlich bestehen die Reliefstrukturen aus erhabenen Teilen und/oder aus Vertiefungen. Beide können in der Form von durchgehenden, konzentrischen oder spiralförmigen Spurrillen oder als isolierte Hügel und/oder Löcher vorliegen. Außerdem kann die Reliefstruktur eine mehr oder weniger glatte Wellenform haben. Den Spurrillen wird hierbei der Vorzug gegeben. Sie weisen in ihrer Querrichtung eine rechteckig sägezahnartige, eine V-förmige oder eine trapezartige Kontur auf. Ihre Vertiefungen werden im allgemeinen als "grooves" und ihre erhabenen Teile als "land" bezeichnet. Von besonderem Vorteil sind hierbei Spurrillen mit 50 bis 200 nm tiefen und 0,4 bis 0,8 µm breiten "grooves", zwischen denen jeweils ein 1 bis 3 µm breites "land" liegt.

Die erfindungsgemäßen dimensionsstabilen Substrate mit jeweils einer Reliefstrukturen aufweisenden Oberfläche haben weiterhin den besonderen Vorteil, daß sie sich vorzüglich für den Aufbau von Audio-Compact-Disks (CD), audiovisuellen Compact-Disks (CDV), laseroptischen Datenplatten und magneto-optischen Datenplatten mit sogenannter Sandwich-Struktur eignen. Zur Herstellung dieser Sandwich-Struktur werden zwei erfindungsgemäße dimensionstabile Substrate paarweise, exakt zentriert übereinander gelegt und so miteinander verbunden, daß ihre Reliefstrukturen aufweisenden Oberflächen, welche entweder bereits die digitalen Daten beinhalten oder welche laseroptische oder magneto-optische Aufzeichnungsschichten tragen, einander parallel zugekehrt sind, wobei ein gewisser Abstand zwischen diesen beiden Oberflächen herrscht. Dieser Abstand kann in üblicher und bekannter Weise mit Hilfe von Abstandshaltern wie Ringen, Stegen oder kleinen Säulen eingestellt werden. Dabei können diese Abstandshalter auch den Raum zwischen den beiden Reliefstrukturen aufweisenden Oberflächen gasdicht abschließen, sofern sie die Form von Ringen haben. Außerdem können die beiden dimensionsstabilen Substrate in ihrer Mitte durch eine Nabe verbunden sein.

Sowohl die Abstandshalter als auch die Nabe können geeignete Formteile beliebiger stofflicher Zusammensetzung sein. Indes ist es von ganz besonderem Vorteil, wenn sowohl die Abstandshalter und, falls verwendet, die Habe erfindungsgemäße Formteile mit der geeigneten äußeren Form sind. Hierbei kann eine haftfeste Verbindung zwischen den einzelnen erfindungsgemäßen Formteilen mit Hilfe geeigneter Klebstoffe erfolgen. Es ist indes von Vorteil, die erfindungsgemäßen Formteile mit Hilfe der bekannten Technik des Ultraschallschweißens zu verbinden.

Außerdem können zwei dieser erfindungsgemäßen dimensionsstabilen Substrate, welche jeweils eine

Reliefstrukturen aufweisende Oberfläche haben, paarweise exakt übereinandergelegt, zentriert und vollflächig haftfest miteinander verbunden werden, so daß ihre Oberflächen mit den Reliefstrukturen nach außen gekehrt sind.

Die Herstellung der erfindungsgemäßen dimensionsstabilen Substrate erfolgt nach den üblichen, vom Stand der Technik her bekannten formgebenenden Methoden. Von Vorteil ist hierbei die formgebende Verarbeitung der neuen Gemische, aus denen die erfindungsgemäßen dimensionsstabilen Substrate bestehen, durch den Spritzguß. Hierzu werden die neuen Gemische auf Extrudern hergestellt und darin als Schmelze zu einer Spritzgußkammer der geeigneten Form transportiert, worin sie zu den dimensionsstabilen Substraten der gewünschten räumlichen Form spritzgegossen werden. Bei diesem Vorgang wird die Reliefstruktur durch eine in der Spritzgußkammer befindlichen Matrize geeigneter Form in die Oberfläche des betreffenden herzustellenden erfindungsgemäßen dimensionsstabilen Substrats eingepreßt. Üblicherweise wird dieses Verfahren unter Reinraumbedingungen durchgeführt.

Je nach Verwendungszweck werden die erfindungsgemäßen dimensionsstabilen Substrate in unterschiedlicher Weise weiterverarbeitet, wobei sie weitere besondere Vorteile zeigen:

Werden die dimensionsstabilen Träger als Audio-Compact-Disks (CD) und als audiovisuelle Compact-Disks (CDV) verwendet, wird ihre Reliefstrukturen aufweisende Oberfläche im allgemeinen zur besseren Reflektierung der Laserstrahlen mit einem Metallspiegel versehen. Auf diesen Metallspiegel können dann in üblicher und bekannter Weise weitere Schichten wie beispielsweise Schutzschichten aufgebracht werden. Die in dieser Weise erhaltenen Compact-Disks weisen eine besonders hervorragend abgeformte Reliefstruktur auf, welche exakt dem Original auf der Matritze entspricht. Die Compact-Disks weisen deshalb eine besonders niedrige Lesefehlerrate (bit error rate) und ein besonders hohes Signal-Rausch-Verhältnis auf. Sie zeigen daher eine ausgesprochen hohe Wiedergabequalität. Darüber hinaus können diese Compact-Disks aufgrund der Färbung der erfindungsgemäßen dimensionsstabilen Substrate in einfacher Weise visuell identifiziert werden.

Werden die erfindungsgemäßen dimensionsstabilen Substrate als Träger von Aufzeichnungsschichten laseroptischer Datenplatten verwendet, wird ihre Reliefstrukturen aufweisende Oberfläche, gegebenenfalls über übliche und bekannte Zwischenschichten, mit einer üblichen und bekannten, im allgemeinen amorphen, thermisch veränderbaren Aufzeichnungsschicht versehen. Diese amorphe, thermisch veränderbare Aufzeichnungsschicht kann aus beliebigen Stoffen aufgebaut sein. Beispielhaft genannt seien Farbstoffe, Tellur oder Gold.

Bekanntermaßen werden die digitalen Daten in diese amorphe, thermisch veränderbare Aufzeichnungsschicht mit Hilfe eines impulsmodulierten Schreiblaserstrahls eines Strahldurchmessers von 300 bis 2000 nm, einer Strahlleistung von 1 bis 20 mW, einer Impulsdauer von 10 bis 1000 ns und einer Schreibwellenlänge $\lambda$ von 400 bis 1000 nm eingeschrieben. Beim Schreibvorgang wird bekanntermaßen der Schreiblaserstrahl über die Aufzeichnungsschicht hinwegeführt. Daher haben die thermisch veränderten Bereiche der Aufzeichnungsschichten im allgemeinen eine runde oder elliptische Grundform. Je nach stofflicher Zusammensetzung der Aufzeichnungsschicht können die thermisch veränderten Bereiche Löcher sein (ablative Datenaufzeichnung); es können Krater (pits) sein, welche gegebenenfalls einen sauber ausgebildeten Kraterrand (wall) aufweisen (deformative Datenaufzeichnung); es können Bereiche sein, in denen durch die Bestrahlung eine Phasenumwandlung des Materials in eine andere Stoffmodifikation stattgefunden hat (Datenaufzeichnung durch Phasenumwandlung). Oder aber, die Aufzeichnungsschicht kann mit Schichten unterlegt sein, welche sich beim Bestrahlen ausdehnen oder Gase entwickeln, wodurch die Aufzeichnungsschichten lokal gedehnt wird, und wobei sich in der Oberfläche der Aufzeichnungsschicht Reliefstrukturen ausbilden, welche die eingeschriebenen digitalen Daten beinhalten. Die Gase können aber auch in der Aufzeichnungsschicht selbst unter Bildung kleiner lichtstreuender Bläschen freigesetzt werden (vesikulare Datenaufzeichnung). Des weiteren kann in den thermisch veränderten Bereichen eine chemische Reaktion eines Bestandteils oder eine chemische Reaktion zwischen mehreren Bestandteilen der Aufzeichnungsschicht stattgefunden haben, oder die thermisch veränderten Bereiche haben sich über die Vergrößerung oder das Zusammenschmelzen von kleinen Partikeln gebildet.

Unabhängig davon, welche Mechanismen im einzelnen der Bildung von thermisch veränderten Bereichen bei der Bestrahlung mit Schreiblaserstrahlen zugrundeliegen, weisen die thermisch veränderten Bereiche eine andere Reflektivität gegenüber Leselaserstrahlen auf als die thermisch unveränderten Bereiche, was zur Detektion der eingeschriebenen Daten ausgenutzt wird. Zu diesem Zweck wird ein Leselaserstrahl einer Strahlleistung von 0,1 bis 2 mW und einer Wellenlänge $\lambda$ von 400 bis 1000 nm über die Aufzeichnungsschicht hinweggeführt, wobei er senkrecht oder schräg auf diese auftrifft. Beim senkrechten Auftreffen kann er, beim schrägen Auftreffen muß er auf die Aufzeichnungsschicht fokussiert sein. Trifft der Leselaserstrahl beim Überstreichen der Aufzeichnungsschicht auf einen thermisch veränderten Bereich, ändern sich die Eigenschaften des von der Aufzeichnungsschicht reflektierten oder transmittierten Lichts,

was mit Hilfe geeigneter Detektoren nachgewiesen werden kann. Geeignete Geräte, mit deren Hilfe laseroptische Datenplatten beschrieben und gelesen werden können, sind die üblichen und bekannten laseroptischen Plattenlaufwerke.

Da man bei dem laseroptischen Schreiben und Lesen von Daten die Aufzeichnungsschichten vorteilhafterweise durch den dimensionsstabilen Träger hindurch bestrahlt, machen sich hierbei die besonderen vorteilhaften Eigenschaften der erfindungsgemäßen dimensionsstabilen Substrate besonders deutlich bemerkbar: Sie ermöglichen das Bestrahlen von thermisch veränderbaren Aufzeichnungsschichten mit Laserstrahlen der unterschiedlichsten Wellenlänge, wobei deren Intensität entweder gar nicht oder in kaum merklicher Weise beeinträchtigt wird. Darüber hinaus schützen sie UV-lichtempfindliche thermisch veränderbare Aufzeichnungsschichten vor der Zersetzung durch UV-Licht, welche bei längerer Lagerung der laseroptischen Datenplatten am Tageslicht eintreten kann. Die laseroptischen Datenplatten, welche die erfindungsgemäßen dimensionsstabilen Substrate enthalten, weisen demnach eine höhere Lebensdauer, eine bessere Korrosionsbeständigkeit, eine höhere Wiedergabequalität, eine niedrigere Lesefehlerrate und ein höheres Signal-Rausch-Verhältnis auf als laseroptische Datenplatten, welche übliche und bekannte dimensionsstabile Substrate enthalten.

Bei der Verwendung der erfindungsgemäßen dimensionsstabilen Substrate als Träger in magneto-optischen Datenplatten, wird ihre Reliefstrukturen aufweisende Oberfläche mit einer üblichen und bekannten magneto-optischen Aufzeichnungsschicht bedeckt. Hierbei kann es sich um eine senkrecht zu ihrer Oberfläche magnetisierte, amorphe ferrimagnetische Lanthanid-Übergangsmetall-Legierungsschicht handeln. Die in diese Aufzeichnungsschichten eingeschriebenen thermisch veränderten Bereiche haben die Form von Flecken, welche eine der ursprünglichen Richtung entgegengesetzte Magnetisierungsrichtung aufweisen. Sie entstehen beim Erwärmen des amorphen ferrimagnetischen Materials dieser Schichten durch den Schreiblaserstrahl unter dem Einfluß eines anliegenden magnetischen Hilfsfeldes. Durch die Erwärmung nimmt die Koerzitivfeldstärke $H_c$ des amorphen ferrimagnetischen Materials ab. Unterschreitet dabei die Koerzitivfeldstärke $H_c$ bei einer vor dem jeweils verwendeten Material abhängigen kritischen Temperatur die Feldstärke des anliegenden magnetischen Hilfsfeldes, so wird der betreffende Bereich ummagnetisiert. Für das Lesen dieser Bereiche verwendet man linear polarisiertes Licht eines kontinuierlich emittierenden Dauerstrichlasers dessen Lichtleistung nicht dazu ausreicht, das Material über die kritische Temperatur hinaus zu erwärmen. Dieser Laserstrahl wird entweder von der Aufzeichnungsschicht selbst oder von einer hinter ihr angeordneten Reflektionsschicht reflektiert, wobei es zu einer Wechselwirkung zwischen den magnetischen Momenten in der Aufzeichnungsschicht und dem magnetischen Vektor der Laserlichtwelle kommt. Durch diese Wechselwirkung wird die Ebene der Polarisation $\vec{E}$ des reflektierten Laserlichts gegenüber der ursprünglichen Ebene um einen kleinen Winkel gedreht, was man auch als Kerr-Effekt oder als Faraday-Effekt bezeichnet. Diese Drehung der Ebene der Polarisation $\vec{E}$ des reflektierten Laserlichts kann mit Hilfe geeigneter optischer und elektronischer Geräte gemessen und in Signale umgesetzt werden. Da man bei diesen Aufzeichnungsverfahren die magneto-optische Aufzeichnungsschicht üblicherweise durch den dimensionsstabilen Träger hindurch bestrahlt, wobei der für das Lesen der Daten maßgebliche physikalische Effekt, d.i. die Drehung der Polarisationsebene des Laserlichts, klein ist, machen sich hier die besonderen Vorteile der erfindungsgemäßen dimensionsstabilen Substrate in ganz besonders hohem Maße bemerkbar. So haben die magneto-optischen Datenplatten, welche die erfindungsgemäßen dimensionsstabilen Substrate enthalten, eine längere Lebensdauer, eine geringere Korrosionsrate, eine geringere Lesefehlerrate und ein höheres Signal-Rausch-Verhältnis als magneto-optische Datenplatten, welche die üblichen und bekannten dimensionsstabilen Substrate enthalten.

## Beispiele und Vergleichsversuche

Im folgenden werden die besonderen Vorteile der erfindungsgemäßen Formteile anhand von dimensionsstabilen Substraten für laseroptische Datenplatten exemplarisch dargestellt.

Bei den folgenden Beispielen und Vergleichsversuchen wurde die Dicke der einzelnen Aufzeichnungsschichten an rasterelektronenmikroskopischen Aufnahmen bestimmt.

Das Signal-Rausch-Verhältnis in dB wurde in bekannter Weise an laseroptischen Datenplatten ermittelt, welche mit Hilfe eines impulsmodulierten Lasers (Wellenlänge $\lambda$ des emittierten Lichts: 830 nm; Pulsdauer: 500 ns; Lichtleistung: 6 mW) durch das dimensionstabile Substrat hindurch beschrieben worden waren. Hierzu wurden die laseroptischen Datenplatten mit Hilfe eines Dauerstrichlasers (Wellenlänge $\lambda$ des emittierten Lichts: 780 nm; Lichtleistung: 0,5 mW) durch das dimensionsstabile Substrat hindurch gelesen. Der emittierte Leselaserstrahl wurde dabei stets mit der relativen Geschwindigkeit von 4 m/sec über die laseroptischen Datenplatten hinweggeführt. Dabei wurde stets das von den laseroptischen Datenplatten

reflektierte Licht erfaßt und analysiert.

Die Schreibempfindlichkeit (nJ), d.h. die geringste Lichtleistung des Schreiblaserstrahls, ab der einwandfrei lesbare thermisch veränderte Bereiche (bits) in einer Aufzeichnungsschicht resultieren, wurde mit Hilfe üblicher und bekannter Meßmethoden ermittelt.

Der Gelbwert YI (Yellowness Index) der dimensionsstabilen Substrate wurde nach DIN 6167 bestimmt und nach DIN 6174 ausgewertet.

In den folgenden Beispielen und Vergleichsversuchen wurden dimensionsstabile Substrate verwendet, welche auf einer Oberfläche übliche und bekannte, konzentrische Spurrillen enthielten. Deren Vertiefungen werden im allgemeinen als "grooves" und ihre erhabenen Teile im allgemeinen als "land" bezeichnet. Die Spurrillen dienen bekanntermaßen der exakten Führung der Schreib- und Leselaserstrahlen, welche vom laseroptischen Schreib- und Lesekopf des Plattenlaufwerks emittiert werden. Hierzu ist es notwendig, daß die im laseroptischen Kopf vorhandenen Spurlagenservoeinrichtungen scharfe optische Signale erhalten. Diese Signale entstehen durch Beugung des von Datenplatten allgemein zu dem laseroptischen Kopf reflektierten Laserlichts an der Grenze zwischen "groove" und "land", wobei man zur Erfassung und Analyse dieser Lichtsignale die hierfür üblichen und bekannten Photodioden-Anordnungen verwendet. Für das "tracking"-Verhalten d.h. die exakte Führung, insbesondere der Leselaserstrahlen, ist es wesentlich, daß ein hoher optischer Kontrast zwischen "land" und "groove" besteht, weil ansonsten nur schwache und verbreiterte oder gar keine Signale mehr erhalten werden. Letzteres ist oftmals bei beschriebenen laseroptischen Datenplatten der Fall. Dies führt aber zu einem geringen Signal-Rausch-Verhältnis und erschwert generell das Wiederauffinden eingeschriebener Daten. Als Maß für die Güte des "trackings" wird daher im allgemeinen die Breite der Signale sowie der Unterschied in der Intensität des von einem "groove" und von einem "land" zurückreflektierten Laserlichts angenommen. Ein zusätzliches Qualitätskriterium ist, ob dieser Intensitätsunterschied über den gesamten Durchmesser einer laseroptischen Datenplatte hinweg konstant und hoch ist.

Demnach wurde im folgenden das "tracking"-Verhalten der laseroptischen Datenplatten über das Lesen der Materialien mit einem Leselaserstrahl ($\lambda$ = 780 nm; Lichtleistung: 0,5 mW) quer zu den Spurrillen ermittelt. Hierbei wurde über den gesamten Durchmesser der laseroptischen Datenplatten hinweggemessen. Das dabei zurückreflektierte Laserlicht wurde mit Hilfe üblicher und bekannter optischer Anordnungen erfaßt, Photodioden zugeleitet und durch geeignete elektronische Bauelemente in elektrische Signale umgewandelt, deren Höhe als Funktion des Datenplattendurchmessers aufgezeichnet wurde. Es wurde beurteilt, ob die Unterschiede zwischen der Höhe der von den "lands" erhaltenen und der von den "grooves" erhaltenen Signale über den gesamten Durchmesser der laseroptischen Datenplatten hinweg konstant, scharf und hoch waren, wonach das "tracking"-Verhaltens wie folgt benotet wurde:

"Sehr gut"

scharfe Signale; großer Unterschied zwischen den Höhen der "land"- und der "groove"-Signale, welcher über den gesamten Durchmesser hinweg konstant ist;

"Gut"

scharfe Signale; großer Unterschied zwischen den betreffenden Signalhöhen, welche aber über den gesamten Durchmesser hinweg mehr oder weniger stark variiert;

"Unbefriedigend"

breite, verwaschene Signale; geringer Unterschied zwischen den betreffenden Signalhöhen; gegebenenfalls variiert dieser Unterschied auch noch über den gesamten Durchmesser hinweg mehr oder weniger stark; oder

"Schlecht"

kein Unterschied mehr zwischen den betreffenden Signalhöhen.

Um zu prüfen, ob das Beschreiben der laseroptischen Datenplatten eine Veränderung in deren "tracking"-Verhalten zur Folge hatte, wurden sowohl die unbeschriebenen als auch die beschriebenen laseroptischen Datenplatten in der oben erläuterten Weise vermessen und benotet. Ergaben sich hierbei gravierende Unterschiede in der "tracking"-Note einer laseroptischen Datenplatte im unbeschriebenen Zustand einerseits und im beschriebenen Zustand andererseits, so stellte dies einen schwerwiegenden Nachteil dar. War der Notenunterschied von einem sehr guten oder guten Niveau aus ausgehend gering, so belegte dies die besondere Vorteilhaftigkeit der betreffenden laseroptischen Datenplatte.

Die unkorrigierte Lesefehlerrate (bit error rate) wurde in üblicher und bekannter Weise über das Beschreiben der laseroptischen Datenplatte mit einer Standardinformation und dem Lesen der Daten ermittelt, wobei ein Zeitintervall-Analysator ("time interval analyzer", TIA) verwendet wurde. Die laseroptischen Datenplatten wurden zunächst unmittelbar nach dem Beschreiben gelesen (1. Lesen).

Die laseroptischen Datenplatten wurden mehrere Wochen lang am Tageslicht gelagert und hiernach erneut gelesen (2. Lesen), um zu ermitteln, ob durch die Lagerung eine Verschlechterung der Eigenschaften

eingetreten war.

Beispiel 1

Die Herstellung der neuen Zusatzstoffe I; allgemeine Versuchsvorschriften:

Methode A

10 g eines Diphenohydrochinons der allgemeinen Formel V wurden in 100 ml Aceton gelöst. Zur resultierenden Lösung gab man 7 g Silberoxid und 10 g geglühtes Natriumsulfat. Nach dem Abkühlen auf 0 ° C wurde die resultierende Mischung mit 2 ml eines sekundären Amins der allgemeinen Formel $HN(R^4)_2$ - (VIII) oder mit 10 ml einer konzentrierten Lösung eines festen sekundären Amins dieser allgemeinen Formel in Aceton versetzt. Hiernach wurde das resultierende Reaktionsgemisch durchmischt. Nach zehnminütiger bis zweistündiger Standzeit wurde die Lösung der Reaktionsprodukte von den darin enthaltenen, festen anorganischen Bestandteilen abgetrennt und eingedampft. Das so erhaltene Rohprodukt wurde mit Hilfe der Hochdruckflüssigchromatographie (high pressure liquid chromatography, HPLC) in Kombination mit der Massenspektrometrie analysiert. Als HPLC-Säule wurde "Reverse Phase Nucleoside 120-5 C18" von Machery und Nagel verwendet. Als Elutionsmittel diente ein Methanol-Wassergemisch, dessen Wassergehalt während der Elution der auf die Säule aufgebrachten Rohprodukte stetig zunahm. Die Wellenlänge λ des für die Detektion verwendeten UV-Lichts war 420 nm.

Für den neuen Zusatzstoff I-3

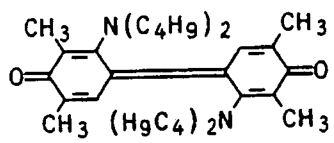

(I - 3)

wurde nach der oben angegebenen Methode eine Retentionszeit von 20,8 Minuten ermittelt. Das Massenspektrum zeigte in Übereinstimmung mit dem theoretisch berechneten Wert einen Mol-Peak bei m/e = 494.

Methode B

15 g eines Diphenochinons der allgemeine Formel IV wurden in 500 ml Toluol suspendiert und mit einer Mischung aus 39 g eines sekundären Amins der allgemeinen Formel $HN(R^4)_2$ (VIII) und 0,72 g CuBr versetzt. Hiernach wurde die Mischung während 3 Stunden unter intensivem Rühren mit einem Sauerstoffstrom von 50 l/h begast, wobei zugleich der Gasraum über der Mischung mit Stickstoff gespült wurde. Nach beendeter Reaktion wurde die so erhaltene Lösung der Reaktionsprodukte von den darin enthaltenen festen Bestandteilen durch Filtration abgetrennt. Hiernach wurde zur Entfernung gelöster Kupferverbindung die Lösung mit einer wäßrigen Lösung des Dinatriumsalzes der Ethylendiamintetraessigsäure extrahiert. Die gereinigte Lösung der Reaktionsprodukte wurde, wie bei Methode A beschrieben, weiterverarbeitet.

Für den neuen Zusatzstoff I - 3, welcher nach der Methode B hergestellt worden war, wurden die gleichen Meßergebnisse erhalten, wie sie bei der Methode A angegeben sind.

Methode C

Die Methode C gleicht weitgehend der Methode B, wobei der einzige Unterschied darin besteht, daß bei der Methode C anstelle des sekundären Amins der allgemeinen Formel $HN(R^4)_2$ (VIII) 5 ml wäßrigen konzentrierten Ammoniaks verwendet wurden.

Für den hierbei resultierenden neuen Zusatzstoff I - 1

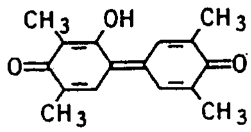

(I - 1)

wurden nach den bei Methode A angegebenen Meßmethoden eine Retentionszeit von 17 Minuten und - in Übereinstimmung mit dem theoretisch berechneten Wert - ein Mol-Peak bei m/e = 256 ermittelt.

Beispiel 2

Die Herstellung der neuen Zusatzstoffe II: allgemeine Versuchsvorschrift:

Die neuen Zusatzstoffe II konnten in sehr einfacher Weise durch Reduktion der Zusatzstoffe I (vgl. das Beispiel 1) mit milden Reduktionsmitteln erhalten werden.

Dazu wurden 20 g eines neuen Zusatzstoffs I mit 20 g $SnCl_2$ $2H_2O$ in 200 ml Aceton vorgelegt. Zu dieser Mischung wurden innerhalb von 5 Minuten 17 ml konzentrierter Salzsäure zugetropft. Nach weiteren 10 Minuten Reaktionszeit wurde die Mischung auf Raumtemperatur abkühlen lassen, und der jeweils resultierende neue Zusatzstoff II wurde durch Zugabe von 1 l Wasser ausgefällt, filtriert, gewaschen und getrocknet.

Die in dieser Weise aus den neuen Zusatzstoffen I - 1 und I - 3 (vgl. Beispiel 1) nahezu quantitativ erhaltenen neuen Zusatzstoff II - 1 und II - 3 wurden IR-spektroskopisch, massenspektroskopisch, massenspektrometrisch, chromatographisch und elementaranalytisch untersucht. Die hierbei erhaltenen Ergebnisse der physikalischen Untersuchungen bestätigten die vorab vermutete Struktur der neuen Zusatzstoffe II - 1 und II - 3, und die Ergebnisse der Elementaranalysen stimmten sehr gut mit den theoretisch berechneten Werten überein.

Beispiel 3

Die Herstellung der neuen Zusatzstoffe III: allgemeine Versuchsvorschrift:

Zu 100 g einer 21,7 %igen wäßrigen Natriumsulfitlösung, welche durch Zugabe von 60,1 g 10 %iger Salzsäure (1,65 mmol) auf pH 6 gebracht worden war, wurden unter Stickstoff 10 g eines sekundären Amins der allgemeinen Formel $HN(R^4)_2$ (VIII) und 10 g eines Diphenochinons der allgemeinen Formel IV gegeben. Die resultierende Mischung wurde mit 200 ml Toluol überschichtet. Anschließend wurde das zweiphasige Reaktionsgemisch während 4 Stunden auf 80°C erhitzt, wobei sich das Diphenochinon IV unter Bildung eines weißen, an der Phasengrenze sich abscheidenden Feststoffs entfärbte. Nach Beendigung der Reaktion hatte die wäßrige Phase einen pH-Wert von 5,7.

Zunächst wurde der weiße Feststoff sowohl von der wäßrigen als auch der organischen Phase abgetrennt. Hiernach wurde die organische Phase von der wäßrigen abgetrennt und eingedampft, wobei eine weitere Feststoff-Fraktion resultierte. Danach wurden die beiden Feststoff-Fraktionen vereinigt. Das in dieser Weise erhaltene Rohprodukt wurde in 60 ml heißem Methanol aufgelöst, wobei geringe Mengen methanolunlöslicher Feststoffe zurückblieben, welche abgetrennt wurden. Aus der methanolischen Lösung kristallisierte beim Abkühlen auf Raumtemperatur zunächst das Diphenohydrochinon der allgemeinen Formel V aus, welches abgetrennt wurde. Die methanolische Mutterlauge wurde im Wasserstrahlvakuum bis zur Trocknung eingeengt, wodurch ein Großteil des Amins der allgemeinen Formel $HN(R^4)_2$ (VIII) entfernt wurde. Der verbleibende Rückstand wurde zur weiteren Abreicherung des Diphenohydrochinons V noch zweimal aus Methanol umkristallisiert. Der neue Zusatzstoff III wurde aus dem in dieser Weise erhaltenen Rohprodukt mit Hilfe der präparativen Säulenchromatographie isoliert.

Nach dieser allgemeinen Vorschrift wurde 3,3′,5,5′-Tetramethyldiphenochinon mit Natriumsulfit in der Gegenwart von Di(n-butyl)amin umgesetzt, wobei 1,6 g des neuen Zusatzstoffs III - 1

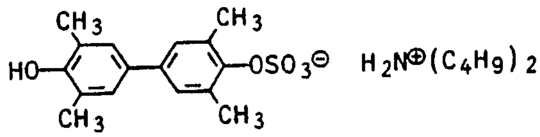

erhalten wurden. Der neue Zusatzstoff III - 1 wurde anhand von Elementaranalysen, welche mit den theoretisch berechneten Werten übereinstimmten, und anhand von kernresonanzspektroskopischen Messungen identifiziert. Die nachfolgende Tabelle 1 gibt einen Überblick über die kernresonanzspektroskopischen Daten des neuen Zusatzstoffs III - 1.

Tabelle 1

| Kernresonanzspektroskopische Daten des neuen Zusatzstoffs III - 1 (24 °C, Tetramethylsilan, in $CDCl_3$) | | |
|---|---|---|
| $^{13}$C-kernresonanzspektroskopische Daten | | |
| Verschiebung | Intensität | Zuordnung |
| 16,2 ppm | 2 | $-CH_3$ |
| 17,6 ppm | 2 | $-CH_3$ |
| 123,8 ppm | 2 | >C= |
| 126,9 ppm | 2 | -CH= |
| 127,0 ppm | 2 | -CH= |
| 132,5 ppm | 1 | >C= |
| 132,7 ppm | 2 | >C= |
| 138,2 ppm | 1 | >C= |
| 148,4 ppm | 1 | >C= |
| 152,1 ppm | 1 | >C= |
| $^{1}$H-kernresonanzspektroskopische Daten | | |
| Verschiebung | Intensität | Zuordnung |
| 2,27 ppm | 6 | $-CH_3$ |
| 2,45 ppm | 6 | $-CH_3$ |
| 7,11 ppm | 2 | -CH= |
| 7,16 ppm | 2 | -CH= |

Beispiele 4 und 5 sowie Vergleichsversuch V1

Herstellung und Eigenschaften erfindungsgemäßer (Beispiele 4 und 5) und nicht erfindungsgemäßer (Vergleichsversuch V1) Formteile bzw. dimensionsstabiler Substrate; allgemeine Versuchsvorschrift:

Für die Beispiele 4 und 5 und den Vergleichsversuch V1 wurden unter exakt vergleichbaren Bedingungen Gemische aus Vinylaromatpolymerisaten und Polyphenylenethern hergestellt. Hierzu wurden in allen Fällen die Vinylaromatpolymerisate auf einem Zweiwellenentgasungsextruder aufgeschmolzen, und der Polyphenylenether wurde zu der jeweiligen Schmelze als 60 %ige Lösung in Toluol hinzudosiert, wobei die Zusatzstoffe zusammen mit den toluolischen Polyphenylenetherlösungen in die Schmelzen eingebracht wurden.

Die resultierenden Gemische wurden auf dem Zweiwellenentgasungsextruder bei einer Verweilzeit von einer Minute unter Entgasung bei 280 °C homogenisiert, aus dem Extruder ausgetragen und zu Granulat verarbeitet.

Die bei 100 °C vorgetrockneten Granulate wurden unter Reinraumbedingungen auf einer 90 to-Spritzgußmaschine und einem Spezialwerkzeug für die Abformung dimensionsstabiler Substrate laseroptischer Datenplatten zu dimensionsstabilen Substraten verspritzt. Hierbei wurden auf einer Seite der dimensionsstabilen Substrate konzentrische Spurrillen mittels einer in das Spritzgußwerkzeug eingelegten Nickelmatrize eingeprägt. Die dimensionsstabilen Substrate waren 1,2 mm dick und hatten einen Durchmesser von 130 mm; Die eingeprägten Spurrillen waren 70 nm tief und 0,6 µm breit ("groove") mit einem Abstand von 1,6 µm ("land").

Für jedes der drei Gemische wurden die optimalen Spritzgußparameter eingestellt, wobei hauptsächlich auf Planarität der dimensionsstabilen Substrate und minimale Doppelbrechung zwischen gekreuzten Polfiltern optimiert wurde. Die Tabelle 2 gibt einen Überblick über die Spritzgußparameter der Gemische und das Eigenschaftsprofil der daraus hergestellten dimensionsstabilen Substrate.

Für die Beispiele 4 und 5 und den Vergleichsversuch V1 wurden die folgenden Gemische verwendet:

Beispiel 4:

30,0 kg radikalisch polymerisiertes Polystyrol mit einem massenmittleren Molekulargewicht $\overline{M}_w$ von $1,65 \times 10^5$,

1,5 kg eines Copolymerisats aus α-Methylstyrol (10 Gew.%) und para-Methylstyrol (90 Gew.%) mit einem massenmittleren Molekulargewicht $\overline{M}_w$ von $9 \times 10^3$,

18,5 kg Poly(2,6-dimethyl-phen-1,4-ylen-ether) der folgenden Spezifikation: Intrinsische Viskosität [η] = 0,545 (gemessen in 0,5 %iger Lösung in Chloroform bei 25° C); zahlenmittleres Molekulargewicht $\overline{M}_n$ von $5,5 \times 10^3$; massenmittleres Molekulargewicht $\overline{M}_w$ von $4,1 \times 10^4$; Uneinheitlichkeit bezüglich des Molekulargewichts ( $\overline{M}_w/\overline{M}_n$) = 7,4;

1,1 g 3,3′,5,5′-Tetramethyldiphenochinon (Zusatzstoff IV);

120 g 3,3′,5,5′-Tetramethyldiphenohydrochinon (Zusatzstoff V);

0,3 g Oligo(2,6-dimethyl-phen-1,4-ylen-ether) eines Oligomerisationsgrades von 3 bis 10 (Zusatzstoffe VI);

4 g Zusatzstoff I - 3

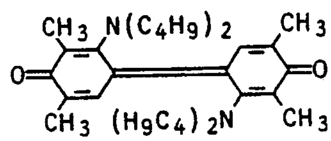 ;

2,1 g Zusatzstoff III - 1

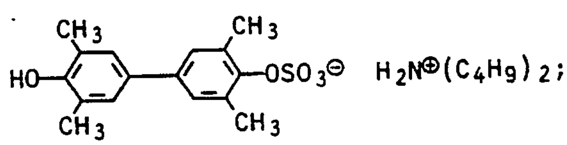 und

60 g p-tert.-Butylkresol.

Beispiel 5:

30,0 kg Polystyrol wie bei Beispiel 4;

1,5 kg eines Copolymerisats aus α-Methylstyrol und p-Methylstyrol wie bei Beispiel 4;

18,5 kg Poly(2,6-dimethyl-10-1,4-ylen-ether) der folgenden Spezifikation: Intrinsische Viskosität [η] = 0,5 (gemessen in 0,5 %iger Lösung in Chloroform bei 25° C); zahlenmittleres Molekulargewicht $\overline{M}_n$ von $1,65 \times 10^4$; massenmittleres Molekulargewicht $\overline{M}_w$ von $3,25 \times 10^4$; Uneinheitlichkeit bezüglich des Molekulargewichts ( $\overline{M}_w/\overline{M}_n$) = 2,0;

0,3 g Zusatzstoff IV gemäß Beispiel 4;

11 g Zusatzstoff V gemäß Beispiel 4;

4,5 g Zusatzstoff I - 3;

25 g Zusatzstoff I - 1

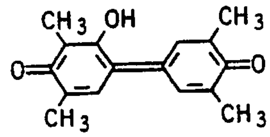 ;

3,4 g Zusatzstoff III - 1;
60 g p-tert.-Butylkresol.

Vergleichsversuch V1:

31,5 kg Polystyrol wie bei Beispiel 4;
18,5 kg Poly(2,6-dimethyl-phen-1,4-ylen-ether) der Spezifikation des Beispiels 5;
0,3 g Zusatzstoff IV gemäß Beispiel 4;
11,5 g Zusatzstoff V gemäß Beispiel 4; und
60 g p-tert.-Butylkresol.

Tabelle 2

Spritzgußparameter der Gemische und Eigenschaftsprofile der daraus hergestellten dimensionsstabilen Substrate

| Spritzgußparameter und Eigenschaften der Substrate | | Bsp. 4 | Bsp. 5 | Vgl.Versuch V1 |
|---|---|---|---|---|
| Schmelzflußindex MFI nach DIN 53 735 der Gemische bei 300°C und einer Auflagekraft von 5 kp | (g/10min) | 96 | 83 | 67 |
| intrinsische Viskosität [η] der Gemische (0,5 %, $CHCl_3$, 25°C) | (dl/g) | 0,734 | 0,689 | 0,695 |
| Einspritzgeschwindigkeit der Gemische | (mm/sec) | 120 | 105 | 90 |
| optimale Einspritztemperatur für die Gemische | (°C) | 330 | 335 | 350 |
| Gelbwert (YI) der Substrate | | 70 | 65 | 15 |
| Maximale Doppelbrechnung der Substrate im Radiusbereich 25 bis 60 mm | (nm) | 2 | 2 | 20 |
| Spurrillenabformung auf der Oberfläche der Substrate (visuelle Begutachtung) | | sehr gut | sehr gut | befriedigend |

Tabelle 2 - Fortsetzung

| | | | | |
|---|---|---|---|---|
| Planlage der Substrate | | sehr gut | sehr gut | befriedigend |
| Maximale Dauergebrauchs-temperatur der Substrate | (°C) | 125 | 125 | 125 |
| Durchlässigkeit für Licht der Wellenlänge λ: | | | | |
| <400 nm | | nicht durch-lässig | nicht durch-lässig | erst ab 370 nm nicht durch-lässig |
| >400 nm | | durchlässig | durchlässig | durchlässig |

Wie der Vergleich zeigt, sind bei den erfindungsgemäßen dimensionsstabilen Substraten der Beispiele 4 und 5 viele Verbesserung gegenüber denjenigen des Vergleichsversuchs V1 eingetreten: die Erniedrigung der Schmelzviskosität führt zur Senkung der Einspritztemperatur und zu verringerter thermischer Schädigung der Gemische. Weiterhin wird die Abformung im Werkzeug deutlich verbessert, ohne daß die maximale Dauergebrauchstemperatur abgesenkt wird. Hervorzuheben ist besonders die Verringerung der Orientierungsdoppelbrechung auf fast nicht mehr meßbare Werte. Auch hinsichtlich des Gelbwertes (YI) der Substrate ergeben sich Vorteile für die erfindungsgemäßen Substrate. Darüber hinaus sind die erfindungsgemäßen Substrate bereits für Licht der Wellenlänge λ = 400 nm nicht mehr durchlässig, wogegen das bekannte dimensionsstabile Substrat das schädigende kurzwellige Licht erst ab λ = 370 nm ausfiltert. Innerhalb der Beispiele 4 und 5 selbst ergibt sich noch einmal eine Differenzierung hinsichtlich des Schmelzflußindices MFI, der Einspritzgeschwindigkeit und der optimalen Einspritztemperatur der Gemische sowie des Gelbwertes (YI) der erfindungsgemäßen dimensionsstabilen Substrate, woraus sich ableiten läßt, daß das erfindungsgemäße Gemisch und Substrat des Beispiels 4 ein Optimum und damit noch weiter vom Stand der Technik entfernt ist als das ohnehin schon vorteilhafte erfindungsgemäße Gemisch und Substrat des Beispiels 5. Daneben wurden bei den Substraten des Vergleichsversuchs V1 die Bildung von vernetzten Gelteilchen beobachtet. Außerdem wiesen die Substrate des Vergleichsversuchs V1 in ihrer Oberfläche bei schräg einfallendem Licht einen perlmuttartigen Schimmer auf, welcher im Hinblick auf die Verwendung dieser Substrate in laseroptischen und noch mehr in magneto-optischen Datenplatten von Nachteil ist.

Beispiele 6 und 7 sowie Vergleichsversuch V2

Herstellung und Eigenschaften laseroptischer Datenplatten mit erfindungsmäßen (Beispiele 6 und 7) und bekannten (Vergleichsversuch V2) dimensionsstabilen Substraten; allgemeine Versuchsvorschrift:

Bei 3000 Umdrehungen/Minute wurden auf einer Schleuderbeschichtungsanlage jeweils zwei der dimensionsstabilen Substrate aus den Beispielen 4 und 5 (für die Beispiele 6 und 7) sowie zwei bekannte dimensionsstabile Substrate aus dem Vergleichsversuch V1 (für den Vergleichsversuch V2) mit propanolischen Lösungen beschichtet, welche jeweils, bezogen auf die Lösung, 4 Gew.% einer Mischung aus einem bei den Wellenlängen λ von 700 bis 850 nm absorbierenden Farbstoff und einem Lösungspolymerisat aus Methylmethacrylat und Methacrylsäure enthielten.

Das Gewichtsverhältnis von Farbstoff zu Polymerisat war 70 : 30. Als Farbstoff wurde ein üblicher und bekannter Trichinocyclopropanfarbstoff verwendet. Nach dem Trocknen wurden laseroptische Datenplatten mit c.a. 100 nm dicken, amorphen, thermisch veränderbaren Aufzeichnungsschichten erhalten, deren Reflektivität für Licht der Wellenlänge λ = 780 nm in bekannter Weise zu 19 % bestimmt wurde (Messung durch die dimensionsstabilen Substrate hindurch).

Jeweils zwei gleiche laseroptische Datenplatten wurden mit den Farbstoffschichten nach innen sandwichartig über einen Abstandsring von 300 µm Dicke als Abstandshalter mittels eines Acrylatklebers haftfest miteinander verbunden.

Die hierbei verwendeten Abstandsringe selbst wurden durch Spritzgießen derjenigen Gemische hergestellt, aus denen die jeweils verwendeten dimensionsstabilen Substrate bestanden. Demgemäß wurde bei Beispiel 6 das Gemisch gemäß dem Beispiel 4, bei Beispiel 7 das Gemisch gemäß dem Beispiel 5 und bei dem Vergleichsversuch V2 das Gemisch gemäß dem Vergleichsversuch V1 angewendet.

Auf einem Plattenlaufwerk wurden in die "grooves" der laseroptischen Datenplatten digitale Daten eingeschrieben. Anschließend wurden die Daten gelesen (1. Lesen). Die hierbei erhaltenen-Ergebnisse finden sich in Tabelle 3.

Hiernach wurden die laseroptischen Datenplatten während dreieinhalb Wochen bei Tageslicht gelagert und danach erneut gelesen (2. Lesen). Die hierbei erhaltenen Ergebnisse finden sich gleichfalls in Tabelle 3.

Tabelle 3

| Anwendungstechnische Eigenschaften laseroptischer Datenplatten mit erfindungsgemäßen (Beispiele 6 und 7) und nicht erfindungsgemäßen (Vergleichsversuch V2) dimensionsstabilen Substraten | | | |
|---|---|---|---|
| Parameter | Bsp. 6[a)] | Bsp. 7[b)] | Vergl.Vers. V2[c)] |
| "tracking"-Verhalten der unbeschriebenen Datenplatten<br>Schreibempfindlichkeit | sehr gut<br>1-2 nJ | sehr gut<br>1-2 nJ | gut<br>1-2 nJ |
| 1. Lesen: | | | |
| Lesesignal [(d)]<br>Signal-Rausch-Verhältnis (dB)<br>Lesefehlerrate (unkorrigiert)<br>"tracking"-Verhalten | gut<br>46<br>$<10^{-5}$<br>sehr gut | gut<br>45<br>$<10^{-5}$<br>gut | gut<br>46<br>$10^{-5}$<br>unbefriedigend |
| 2. Lesen | | | |
| Lesesignal [(d)]<br>Signal-Rausch-Verhältnis (dB)<br>Lesefehlerrate (unkorrigiert)<br>"tracking"-Verhalten | gut<br>45<br>$<10^{-5}$<br>sehr gut | gut bis befriedigend<br>43<br>$<10^{-5}$<br>gut | befriedigend<br>32<br>$>10^{-5}$<br>unbefriedigend |

a) dimensionsstabiles Substrat aus Beispiel 4;
b) dimensionsstabiles Substrat aus Beispiel 5;
c) dimensionsstabiles Substrat aus Vergleichsversuch V1;
d) Kriterium ist sowohl die Höhe (Stärke) als auch die Breite des durch einen thermisch veränderten Bereich (bit) hervorgerufenen Lesesignals; Bestimmung mit einem Oszilloskop; Benotung: "Gut" (sch rfe starke Signale); "Befriedigend" (etwas verbreiterte starke Signale); "Unbefriedigend" (sehr breite und/oder schwache Signale);

Die Ergebnisse lassen sich wie folgt zusammenfassen:

Während die weitgehend durch die Farbstoffschicht selbst bedingten anwendungstechnischen Eigenschaften wie Schreibempfindlichkeit und Lesesignal von den unterschiedlichen dimensionsstabilen Substraten nicht oder nur wenig beeinflußt wurden, konnte bei den laseroptischen Datenplatten mit den erfindungsgemäßen Substraten (Beispiele 6 und 7) beim ersten Lesen eine deutliche Verbesserung des Signal-Rausch-Verhältnisses, der unkorrigierten Lesefehlerrate und insbesondere der Spurführung gegenüber dem Stand der Technik beobachtet werden: Die beschriebenen Teile der laseroptischen Datenplatten mit den bekannten Substraten konnten kaum gelesen werden, wogegen bei den laseroptischen Datenplatten mit den erfindungsgemäßen Substraten die Spurführung mühelos einzuhalten war, weswegen sich denn auch hier die eingeschriebene Information so gut wie fehlerfrei wiedergewinnen ließ.

Die beim 2. Lesen der laseroptischen Datenplatten erhaltenen Ergebnisse machen deutlich, wie stark die stoffliche Zusammensetzung der jeweils verwendeten dimensionsstabilen Substrate deren anwendungstechnischen Eigenschaften bestimmt: So wiesen die laseroptischen Datenplatten mit erfindungsgemäßen Substraten nach längerer Lagerung am Tageslicht noch immer gute bis sehr gute anwendungstechnische Eigenschaften auf, wogegen die laseroptischen Datenplatten mit den bekannten Substraten erhebliche Mängel aufwiesen, was die besondere Vorteilhaftigkeit der erfindungsgemäßen dimensionsstabilen Substrate weiter untermauert.

## Ansprüche

1. Optisch klares, isotropes, von Orientierungsdoppelbrechung freies Formteil für optische Zwecke aus einem Gemisch aus Polyphenylenethern, Vinylaromatpolymerisaten und Zusatzstoffen, dadurch gekennzeichnet, daß das Gemisch, bezogen auf seine Gesamtmenge, 1 bis 1000 ppm mindestens eines Zusatzstoffes der allgemeinen Formel I,

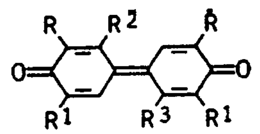

(I)

und/oder der allgemeinen Formel II,

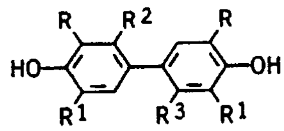

(II)

enthält, worin die Reste R und $R^1$ Wasserstoff-, Chlor- oder Bromatome, substituierte oder unsubstituierte $C_1$- bis $C_6$-Alkylgruppen, $C_3$- bis $C_6$-Alkenylgruppen, substituierte oder unsubstituierte $C_6$- bis $C_{18}$-Arylgruppen oder $C_1$- bis $C_6$-Alkoxygruppen bezeichnen, wobei die Reste R und $R^1$ gleich oder voneinander verschieden sein können und wobei

a) die Reste R für Wasserstoffatome oder Methylgruppen stehen, sofern die Reste $R^1$ Chlor- oder Bromatome bezeichnen,

b) die Reste R für Methylgruppen stehen, sofern die Reste $R^1$ Isopropylgruppen bezeichnen und wobei

c) tert.-Butylgruppe ausgenommen ist;

und worin die Reste $R^2$ und $R^3$ Wasserstoffatome, Hydroxygruppen oder $N(R^4)_2$-Gruppen bezeichnen, wobei

d) der Rest $R^3$ nur dann für ein Wasserstoffatom steht, wenn der Rest $R^2$ eine Hydroxygruppe oder eine $N(R^4)_2$-Gruppe bezeichnet,

e) der Rest $R^3$ (nur dann für eine $N(R^4)_2$-Gruppe steht, wenn der Rest $R^2$ die selbe Bedeutung hat, und

f) die beiden Reste $R^4$ an einem Stickstoffatom gleich oder verschiedenen voneinander sein können und verzweigte, unverzweigte oder cyclische Alkylgruppen oder verzweigte, unverzweigte oder cyclische Aza- oder Oxaalkanylgruppen bezeichnen oder wobei die beiden Reste $R^4$ über eine zweiwertige Gruppe cyclisch miteinander verknüpft sind.

2. Das Formteil für optische Zwecke nach Anspruch 1, dadurch gekennzeichnet, daß das Gemisch, bezogen auf seine Gesamtmenge, mindestens einen weiteren Zusatzstoff der allgemeinen Formel III,

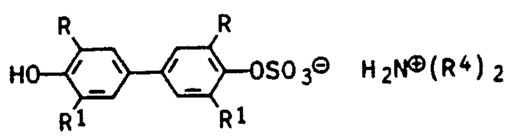

(III)

in einer Menge von 1 bis 1000 ppm enthält, wobei die Reste R, $R^1$ und $R^4$ die in Anspruch 1 angegebene Bedeutung haben.

3. Das Formteil für optische Zwecke nach Anspruch 1 oder 2, dadurch gekennzeichnet, daß das Gemisch, bezogen auf seine Gesamtmenge, 1 bis 10.000 ppm mindestens eines weiteren Zusatzstoffes der allgemeinen Formel IV

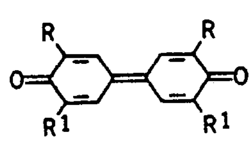

(IV)

und/oder der allgemeinen Formel V

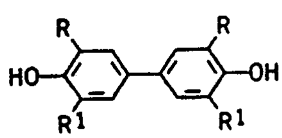

(V)

enthält, worin die Reste R und $R^1$ die in Anspruch 1 angegebene Bedeutung haben.

4. Das Formteil für optische Zwecke nach einem der Ansprüche 1 bis 3, dadurch gekennzeichnet, daß das Gemisch, bezogen auf seine Gesamtmenge bis 10.000 ppm an oligomeren Phenylenethern der allgemeinen Formel VI

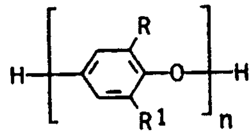 (VI)

enthält, worin die Reste R und $R^1$ die in Anspruch 1 angegebene Bedeutung haben und worin n eine ganze Zahl von 3 bis 15 ist.

5. Das Formteil für optische Zwecke nach einem der Ansprüche 1 bis 4, dadurch gekennzeichnet, daß die Reste R und $R^1$ Methylgruppen bezeichnen.

6. Das Formteil für optische Zwecke nach einem der Ansprüche 1 bis 5, dadurch gekennzeichnet, daß die Reste $R^4$ n-Butylgruppen bezeichnen.

7. Das Formteil für optische Zwecke nach einem der Ansprüche 1 bis 6, dadurch gekennzeichnet, daß das Gemisch für Licht einer Wellenlänge $\lambda$ <400 nm undurchlässig und für Licht einer Wellenlänge $\lambda$ >400 nm durchlässig ist.

8. Das Formteil für optische Zwecke nach einem der Ansprüche 1 bis 7, dadurch gekennzeichnet, daß das Gemisch einen Gelbwert (YI, Yellowness Index) nach DIN 6167 von 40 bis 90 hat.

9. Das Formteil für optische Zwecke nach einem der Ansprüche 1 bis 8, dadurch gekennzeichnet, daß das Gemisch eine in 0,5 %iger Lösung in Chloroform bei 25° C gemessene intrinsische Viskosität von 0,5 bis 0,8 aufweist.

10. Das Formteil für optische Zwecke nach einem der Ansprüche 1 bis 9, dadurch gekennzeichnet, daß die Polyphenylenether und die Vinylaromatpolymerisate in einem Gewichtsverhältnis von 30 : 70 bis 50 : 50 in dem Gemisch vorliegen.

11. Das Formteil für optische Zwecke nach Anspruch 10, dadurch gekennzeichnet, daß die Polyphenylenether und die Vinylaromatpolymerisate in einem Gewichtsverhältnis von 35 : 65 bis 40 : 60 in dem Gemisch vorliegen.

12. Das Formteil für optische Zwecke nach einem der Ansprüche 1 bis 11, dadurch gekennzeichnet, daß die Polyphenylenether eine in 0,5 %iger Lösung in Chloroform bei 25° C gemessene intrisische Viskosität $[\eta]$ von 0,5 bis 0,6 aufweisen.

13. Formteil für optische Zwecke( nach einem der Ansprüche 1 bis 12, dadurch gekennzeichnet, daß die Polyphenylenether eine Uneinheitlichkeit bezüglich des Molekulargewichts $\overline{M}_w/\overline{M}_n$ von 4 bis 11 haben.

14. Das Formteil für optische Zwecke nach Anspruch 13, dadurch gekennzeichnet, daß die Polyphenylenether ein zahlenmittleres Molekulargewicht $\overline{M}_n$ von 3500 bis 7000 haben.

15. Das Formteil für optische Zwecke nach Anspruch 13 oder 14, dadurch gekennzeichnet, daß die Polyphenylenether ein massenmittleres Molekulargewicht $\overline{M}_w$ von 30.000 bis 50.000 haben.

16. Das Formteil für optische Zwecke nach einem der Ansprüche 1 bis 15, dadurch gekennzeichnet, daß es sich bei den Polyphenylenethern um Poly(2,6-dimethyl-phen-1,4-ylen-ether) handelt.

17. Das Formteil für optische Zwecke nach einem der Ansprüche 1 bis 16, in der Form einer runden gelochten Scheibe mit zwei planaren Oberflächen.

18. Das Formteil für optische Zwecke nach einem der Ansprüche 1 bis 16, in der Form einer runden gelochten Scheibe mit mindestens einer Oberfläche, welche Reliefstrukturen im Mikrometer- und/oder Submikrometerbereich aufweist.

19. Verwendung des Formteils gemäß Anspruch 17 oder 18 als Träger für digitale oder analoge Daten.

20. Verwendung des Formteils gemäß Anspruch 17 oder 18, als dimensionsstabiles Substrat für laseroptische und magneto-optische Aufzeichnungsschichten sowie für Audio-Compact-Disks (CD) und audiovisuelle Compact-Disks (CDV).

21. Audio-Compact-Disk (CD), audiovisuelle Compact-Disk (CDV), laseroptische Datenplatte und magneto-optische Datenplatte, dadurch gekennzeichnet, daß sie Formteile gemäß Anspruch 17 oder 18 als dimensionsstabile Substrate enthalten.

22. Audio-Compact-Disk (CD), audiovisuelle Compact-Disk (CDV), laseroptische Datenplatte oder magneto-optische Datenplatte mit Sandwich-Struktur, gebildet von zwei exakt übereinander angeordneten, zentrierten dimenssionsstabilen Substraten, welche in ihrem Randbereich und/oder im Bereich ihrer Mitte über Abstandshalter haftfest miteinander verbunden sind, dadurch gekennzeichnet, daß es sich bei den

beiden dimensionsstabilen Substraten um zwei Formteile gemäß Anspruch 17 oder 18 handelt und die Abstandshalter in geeigneter Weise geformte Formteile gemäß einem der Ansprüche 1 bis 16 sind.

23. Audio-Compact-Disk (CD), audiovisuelle Compact-Disk (CDV), laseroptische Datenplatte oder magneto-optische Datenplatte mit zwei exakt übereinander angeordneten, zentrierten und vollflächig haftfest miteinander verbundenen dimensionsstabilen Substraten, dadurch gekennzeichnet, daß es sich bei den beiden Substraten um zwei Formteile gemäß Anspruch 17 oder 18 handelt.

24. Schwefelsäurehalbester von Hydrochinonen der allgemeinen Formel III,

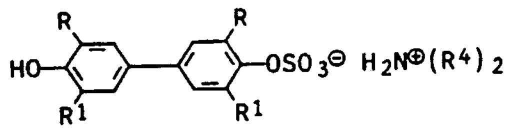

(III)

worin die Reste R und $R^1$ für Methylgruppen stehen und die Reste $R^4$ die in Anspruch 1 angegebene Bedeutung haben.

25. Diphenochinone der allgemeinen Formel I,

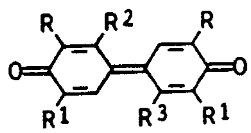

(I)

worin die Reste R und $R^1$ für Methylgruppen stehen und die Reste $R^2$ und $R^3$ die in Anspruch 1 angegebene Bedeutung haben.

26. Diphenohydrochinone der allgemeinen Formel II,

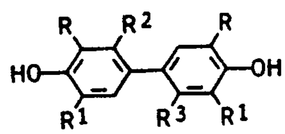

(II)

worin die Reste R und $R^1$ für Methylgruppen stehen und die Reste $R^2$ und $R^3$ die in Anspruch 1 angegebene Bedeutung haben.